# NEW EUROPEAN PATENT SPECIFICATION

(11) **EP 1 131 447 B2**
(45) Date of publication and mention of the opposition decision: **12.10.2011**
(45) Mention of the grant of the patent: 08.11.2006
(21) Application number: 99972259.8
(22) Date of filing: 16.11.1999
(51) Int. Cl.: C12N 15/56, C12N 9/24, A23K 1/165

(54) **THERMOSTABLE XYLANASES**
HITZESTABILE XYLANASEN
XYLANASES THERMOSTABLES

(30) Priority: 16.11.1998 US 108504 P
(43) Date of publication of application: 12.09.2001
(73) Proprietor: NATIONAL RESEARCH COUNCIL OF CANADA, Ottawa, Ontario K1A OR6 (CA)
(72) Inventor: SUNG, Wing, L., National Research Coucil Canada, Ottawa, Ontario K1A 0R6 (CA); TOLAN, Jeffrey, S., Ottawa, Ontario K1V 1C1 (CA)
(74) Representative: Caldwell, Judith Margaret
(86) International application number: PCT/CA1999/001093
(87) International publication number: WO 2000/029587

(56) References cited:
- EP-A- 0 828 002
- EP-A2- 0 828 002
- WO-A-94/24270
- WO-A-95/29997
- WO-A1-94/24270
- WAKARCHUK W W ET AL: "THERMOSTABILIZATION OF THE BACILLUS CIRCULANS XYLANASE BY THE INTRODUCTION OF DISULFIDE BONDS" PROTEIN ENGINEERING,GB,OXFORD UNIVERSITY PRESS, SURREY, vol. 7, no. 11, 1 January 1994 (1994-01-01), pages 1379-1386, XP002072553 ISSN: 0269-2139 cited in the application
- MOREAU A ET AL: "INCREASE IN CATALYTIC ACTIVITY AND THERMOSTABILITY OF THE XYLANASE A OF STREPTOMYCES LIVIDANS 1326 BY SITE-SPECIFIC MUTAGENESIS" ENZYME AND MICROBIAL TECHNOLOGY,US,STONEHAM, MA, vol. 16, no. 5, 1 May 1994 (1994-05-01), pages 420-424, XP002072550 ISSN: 0141-0229
- GRUBER K ET AL: "Thermophilic xylanase from Thermomyces lanuginosus: high-resolution X-ray structure and modeling studies" BIOCHEMISTRY, vol. 37, no. 29, 29 September 1998 (1998-09-29), pages 13475-13485, XP002131131 EASTON, PA US
- AY ET AL PROC.NATL.ACAD. SCI USA 01 June 1998, pages 6613 - 6618

## Description

The present invention relates to thermostable xylanase enzymes. More specifically, the present invention is directed to thermostable xylanase enzymes that exhibit high activity at or near physiological pH and temperature, and their use in feed pelleting applications.

### BACKGROUND OF THE INVENTION

Natural xylanase enzymes, such as that of the fungus *Trichoderma reesei,* have been added to animal feed to increase the efficiency of digestion and assimilation of nutrients. During digestion of feed grains such as wheat and barley, non-starch polysaccharides, including xylan, increases the viscosity of the digesta in the absence of added exogenous enzyme. This interferes with the diffusion of the digestive enzymes to the feed and the subsequent assimilation of the nutrients. The highly viscous digesta increases the occurrence of sticky stool, which increases the likelihood of disease and causes effluent run-off problems. The addition of xylanase in feed breaks down the xylan and decreases the viscosity of the digesta, thereby helping tp alleviate these problems. Xylanase produces a cost saving by increasing the efficiency of feed conversion. Xylanase can decrease the feed consumed/ weight gain ratio by 5-15% (Viveros, A., Brenes, A., Pizarro, M. and Castano, M., 1994, Animal Feed Sci. Technol. 48:237-251).

Xylanase enzymes used for feed are typically aqueous solutions of active protein, stabilizers, preservatives and other additives. The enzymes are typically sprayed onto the feed at concentration of 100-2000 ml per tonne feed. Alternatively, granular or powdered xylanase can be used. Once the feed is consumed by the animal, the enzyme acts on xylan as the feed is ingested and digested in the gut. Eventually the xylanase, a protein molecule, is hydrolysed by the digestive enzymes (proteases) into amino acids like any protein in the feed.

Increasingly, animal feeds are pelleted at high temperatures for sterilization against harmful bacteria, for example *Salmonella.* Feed pelleting is carried out by heating the feed solids with 100 to 140°C steam and passing them through an extruder/pelleting auger to form feed pellets, which then cool in a storage bin. The typical time required for the material to pass through the system is 30 minutes. As is known in the art, higher temperatures can be used with shorter pelleting times, and lower temperatures with longer pelleting times, provided that the necessary moisture levels are obtained. The overall resulting temperature within the solids, prior to, during, and after pellet formation reaches about 70-95 °C, for up to 30 min. It is desirable to add the xylanase during the feed pelleting process. This would save the feed formulators the additional step of adding liquid xylanase, which is inconvenient and can introduce microbial contamination into the feed. The option of adding solid xylanase as a separate step is also undesirable, as the solids would not be evenly mixed. Marquardt and Bedford (1997. Enzymes in Poultry and Swine Nutrition, Marquardt R.R. and Han Z. eds., pp.129-138) indicate that even though currently available enzymes are beneficial for use as feed additives, new enzymes exhibiting high activity and resistance to heat treatment are also desired, however, they note that enzymes exhibiting these properties are not available.

Xylanases of Family 11 (also termed Family G xylanases) have several properties suitable for feed applications due to their small size and high activity. An example of a moderate temperature Family 11 xylanases is TrX, which is obtained from *Trichoderma reesei.* Moderate temperature xylanases are proven feed additive enzymes with temperature and pH optima compatible with the physiological conditions in the digestive system of animals. However, these enzymes can not tolerate the high temperature of the pelleting process and become inactive during this step.

Xylanases from high temperature microorganisms (eg. a thermophile), for example *Thermomonospora fusca* xylanase (termed TfX, also a Family 11 xylanase), have also been considered for feed pelleting. The thermostability of such enzymes is sufficient to tolerate the pelleting temperatures. However, thermophilic xylanases have optimum activity at high temperatures (70-80°C), and several of these enzymes have a high pH optimum of 7-9. When introduced into the digesting system of an animal, with a physiological temperature of around 40°C (e.g. poultry 43°C, a similar temperature is noted within swine) and pH of 3-5 in the digesta, these enzymes function poorly.

Family 11 xylanases have been modified by protein engineering to improve the properties of these enzymes for industrial applications. These modifications have been directed at increasing the temperature and pH optima, along with the thermostability, of these enzymes for specific applications. For example, US 5,405,769 (WO 94/24270) is directed to site-specific mutagenesis of *Bacillus circulans* xylanase (BcX) for the improvement of the thermostability of this enzyme. The disclosed modifications relate to the formation of intermolecular and intramolecular disulfide bonds within BcX, and these modifications resulted in increased thermostability. For example, an improvement in thermostability of up to 6°C with the addition of a single disulfide bond, and up to 10°C with two disulphide bonds was observed. Other modifications included linking the N- and C- termini which increased thermostability by 6°C, or N-terminal mutations, which increased thermostability by 2°C. However, with all of the above modifications the resultant enzymes were either less active (up to 45% less active), or exhibited an increase in the temperature and pH optima. As such these enzymes are not suitable for feed pelleting applications.

US 5,759,840 also discloses modifications to BcX and *Trichoderma reesei* xylanase (TrX) to increase the thermostability; while at the same time increase the temperature and pH optima of these enzymes. Again, these xylanases would not be suitable for feed pelleting applications.

The above results are in agreement with other reports that note that disulfide bonds are not among the thermostabilization mechanisms employed by thermophilic enzymes (Cowan, D.A., 1995, Essays Biochem. 29:193-207), as the disulfide can be broken into dehydroalanine and thiocysteine at temperatures over 80°C. Therefore, the enhancement of stability of an enzyme using disulfide bonds is limited to lower temperature ranges. The disulfide bond is thus not recommended to improve the stability of the enzyme at high temperatures (Gupta, M. N. , 1991, Biotech. Applied Biochem. 14:1-11; Cowan, D.A., 1995, Essays Biochem. 29:193-207. ).

None of the above documents address methods for obtaining xylanase enzymes using conventional screening techniques, or by modifying xylanase enzymes, that exhibit the properties of higher temperature tolerance while maintaining optimal performance under conditions of physiological pH and temperature.

An improvement in the thermostability of *Trichoderma reesei* xylanase II was reported by Paloheimo et al (Paloheimo, M., Mantyla, A., Vehmaanpera, J., Hakola, S., Lantto, R. , Lahtinen, T., Parkkinen, E., Fagerstrom, R. and Suominen, P. 1997, in Carbohydrases from Trichoderma reesei and Other Microorganisms p255-264). Of the five mutants characterized, the most improved mutant (glutamic acid-38 TrX) retained 50 % of activity at 57°C after 9 min, as compared to 7 min by wide type TrX. Arase et al (Arase, A., Yomo, T., Urabe, I., Hata, Y., Katsube, Y. and Okada, H., 1993, FEBS Lett. 316:123-127) describes several modifications to improve the thermostability of a *Bacillus pumilis* xylanase (BpX), however only up to 40% of the residual enzymatic activity was maintained following incubation of these enzymes at a temperature of 57°C for 20 min. Even though, in both of these studies the effects of increased thermostability on pH and temperature optima of the enzymes were not determined, these enzymes exhibit inadequate thermostability for feed pelleting applications.

In spite of a wide range of experience in screening, testing and modifying xylanase enzymes, there are no reports of xylanases that exhibit the combination of properties required for feed pelleting applications: high thermostability, with optimum activity at physiological pH and temperature. No natural xylanases have been selected, nor has any mutation methodology for the Family 11 xylanases been developed to increase thermostability of xylanase enzymes to, without any change in the temperature and pH optima, and a concomitant loss of the specific activity of the enzyme. Such selected natural xylanases, or xylanases prepared using mutation methodology would offer the advantage of enhancement of feed digestibility and processing in pelleting.

The present invention is directed to obtaining xylanase enzymes that exhibit the property of increased thermostability, while maintaining the pH and temperature optima that are typically found under physiological conditions.

It is an object of the invention to overcome disadvantages of the prior art.

The above object is met by the combinations of features of the main claims, the sub-claims disclose further advantageous embodiments of the invention.

### SUMMARY OF THE INVENTION

The present invention relates to thermostable xylanase enzymes. More specifically, the present invention is directed to thermostable xylanase enzymes that exhibit high activity at or near physiological pH and temperature, and the use of these xylanase enzymes in feed pelleting applications.

According to the present invention there is provided an isolated modified Family 11 Trichoderma, Streptomyces or Schizophyllum xylanase, comprising at least one intramolecular disulfide bond, and a substituted basic amino acid at position 162 (*Trichoderma reesei* xylanase II numbering) or its equivalent, said position determined from sequence alignment of said isolated xylanase with the *Trichoderma reesei* xylanase II amino acid sequence defined in SEQ ID NO:16, said isolated xylanase exhibiting at least 40% of optimal activity from about pH 3.5 to about pH 6.0, and from about 40 to about 60°C, and at least 30% of optimal activity after a pre-incubation step for 30 minutes at 70°C, 80°C or 90°C in the presence of 40% glycerol; a pre-incubation step for 30 or 60 minutes at 62.5°C in the absence of a stabilizer; or a pre-incubation step of 30 minutes at 64°C or 68°C in the absence of a stabilizer. The basic amino acid is selected from the group consisting of lysine, arginine and histidine. Preferably, the basic amino acid is histidine.

The modified xylanase of the invention comprises at least one disulfide bridge. Preferably, the modified xylanase comprises one or two disulfide bridges.

The present invention is also directed to the isolated xylanase as defined above wherein said xylanase is selected from the group consisting of TrX-162H-DSI, TrX-162H-DS2, and TrX-162H-DS4.

The present invention also pertains to a method of preparing animal feed, wherein the method comprises applying the isolated, modified, Family 11 xylanase as defined above onto the animal feed to produce a xylanase-animal feed combination, and heat sterilizing the xylanase-animal feed combination. Preferably, the animal feed is a poultry or swine feed.

The present invention is directed to obtaining xylanase enzymes that exhibit pH and temperature optima that are found within the digesta of an animal, while at the same time the xylanase molecule exhibits thermostability and can therefore withstand processes associated with sterilizing and producing pelleted feed. The prior art discloses obtaining thermostable enzymes, either through selection of native enzymes or through genetic engineering, however, these enzymes do not exhibit physiological pH and temperature optima. The prior art also discloses xylanase enzymes that exhibit optimal enzyme activity at physiological pH and temperature, however, these enzymes are not thermally stable. Furthermore, there is nothing in the prior art to suggest that native xylanase enzymes, or that xylanase enzymes may be modified as disclosed herein in order to obtain xylanase enzymes that exhibit high temperature tolerance suitable for feed pelleting, and retain optimum enzymatic activity at or near physiological conditions.

This summary of the invention does not necessarily describe all necessary features of the invention but that the invention may also reside in a sub-combination of the described features.

### BRIEF DESCRIPTION OF THE DRAWINGS

These and other features of the invention will become more apparent from the following description in which reference is made to the appended drawings wherein:

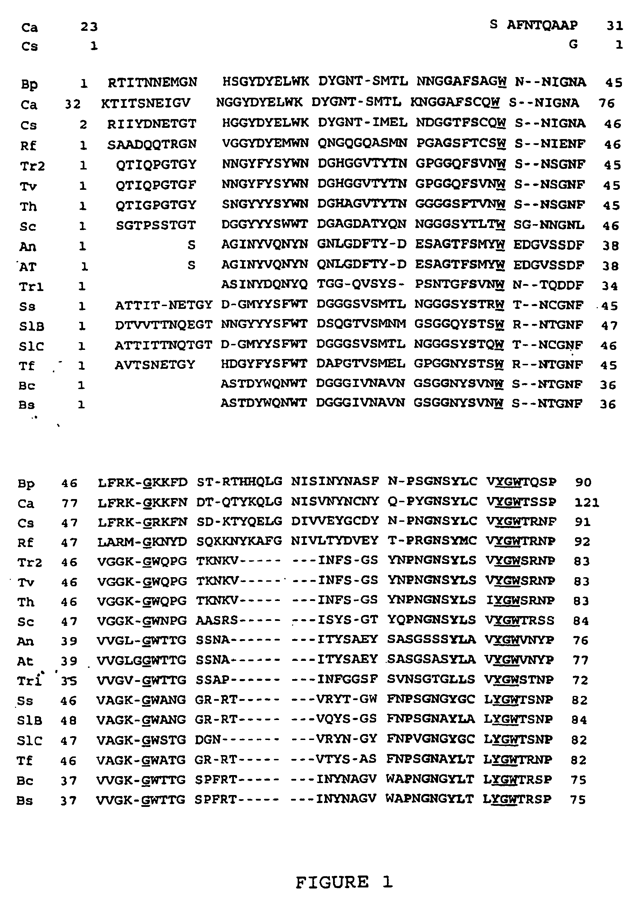

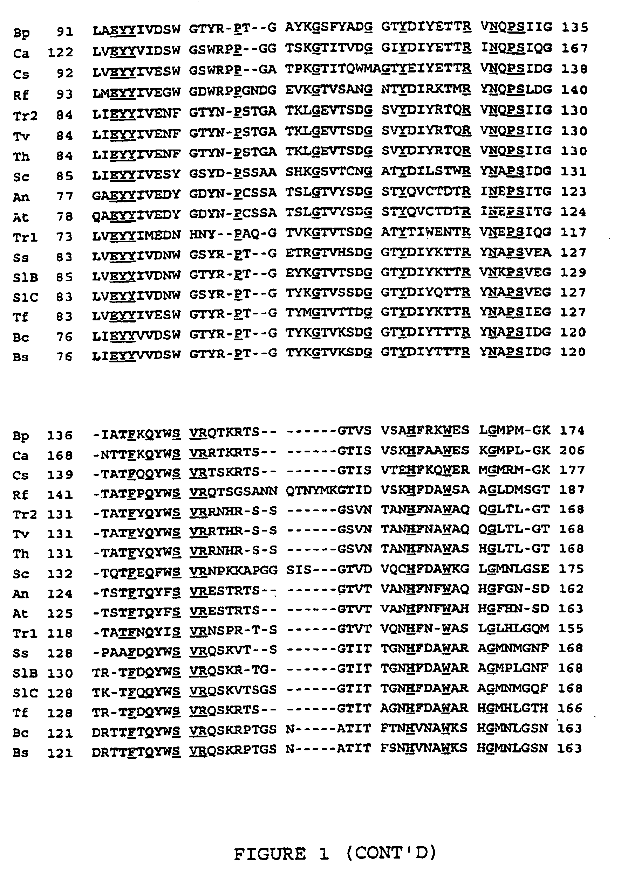

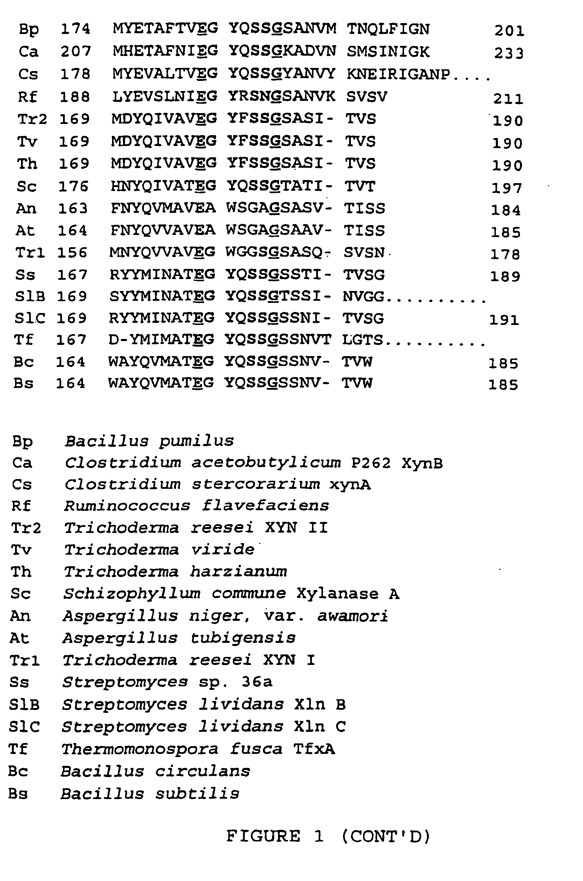

- FIGURE 1: shows the multiple amino acid sequence alignment among Family 11 xylanases. The amino acids common to at least 80% of the Family 11 xylanases listed are indicated in bold. The residues common to all Family 11 xylanases are underlined. *Bacillus pumilus* (Bp); *Clostridium acetobutylicum* P262 XynB (Ca); *Clostridium stercorarium* (Cs); *Ruminococcus flavefaciens* (Rf); *Trichoderma reesei* XynII (Tr2); *Trichoderma viride* (Tv); *Trichoderma harzianum* (Th); *Schizophyllum commune* Xylanase A (Sc); *Aspergillus niger* var. awamori (An); *Aspergillus tubigensis* (At); *Trichoderma reesei* XynI (Tr1); *Streptomyces sp. No. 36a* (Ss); *Streptomyces lividans* Xylanase B (S1B); *Streptomyces lividans* Xln C (S1C); *Thermomonospora fusca* TfxA (Tf); *Bacillus circulans* (Bc); *Bacillus subtilis* (Bs)
- **FIGURE 2**: shows the synthetic oligonucleotides for the construction of gene sequence encoding the *Trichoderma* xylanase in the plasmid pTrX (SEQ ID NO:18).
- **FIGURE 3**: shows the effect of incubation time on the residual enzymatic activity of mutant TrX, TrX-DS1, TrX-162H, TrX-162H-DS1, and TrX-162H-DS4 at 62.5°C. The data are normalized to that observed at 0 min.

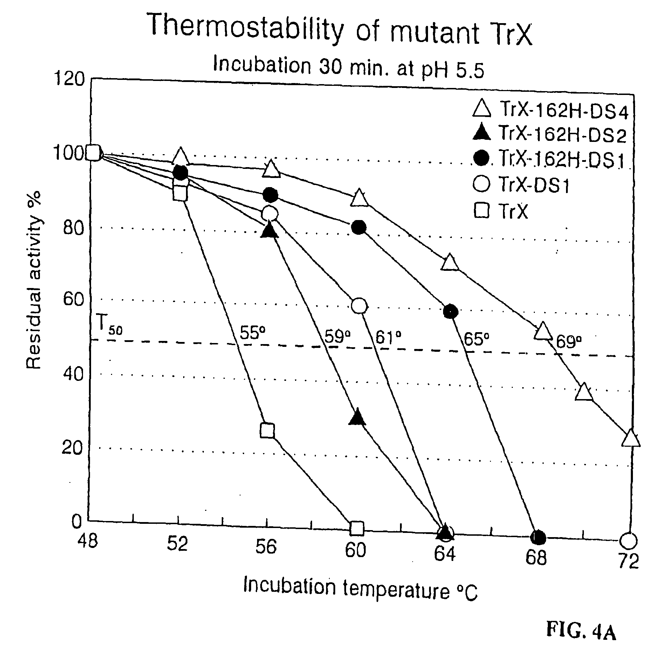

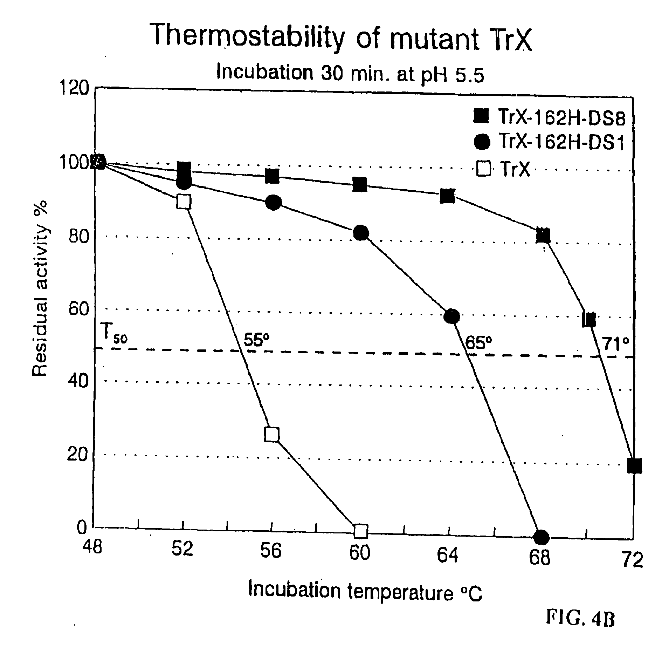

- **FIGURE 4**: shows the effect of temperatures on the residual enzymatic activity of several of the modified xylanases of the present invention. Figure 4(a) shows the residual enzymatic activity of TrX, TrX-DS1, TrX-162H-DS1, TrX-162H-DS2, and TrX-162H-DS4 in sodium citrate buffer in a 30 min incubation. Figure 4(b) shows the effect of temperatures on the residual enzymatic activity of the mutant TrX-DS8. For Figures 4(a) and (b) The data are normalized to that observed at 48°C. The T₅₀, which is the incubation temperature allowing the maintenance of 50% residual activity after 30 min, was determined for each mutant TrX.
- **FIGURE 5**: shows the effect of temperatures on the residual enzymatic activity of mutant TrX, Trx-DS1 and TrX-162H-DS1 in 40% glycerol in a 30 min incubation. The data are normalized to that observed at 50°C.
- **FIGURE 6**: shows the effect of incubation time on the residual enzymatic activity of TrX-162H-DS1 in 40% glycerol at 90°C. The data are normalized to that observed at 0 min.
- **FIGURE 7**: shows the effect of temperature on release of xylose in a 30 min hydrolysis of soluble xylan by TrX, TrX-162H-DS1, TrX-162H-DS2 and TrX-162H-DS4 at pH 4.5. The data are normalized to that observed at the temperature optimum.
- **FIGURE 8**: shows the effect of pH on the release of xylose in a 7 min hydrolysis of soluble xylan by TrX, TrX-162H-DS1, TrX-162H-DS2 and TrX-162H-DS4 at 40°C. The data are normalized to that observed at the pH optimum.

### DESCRIPTION OF PREFERRED EMBODIMENT

The present invention relates to thermostable xylanase enzymes and their use as feed additives. More specifically, the present invention is directed to thermostable xylanase enzymes that show good thermostability and exhibit high activity at or near physiological pH and temperature.

The following description is of a preferred embodiment by way of example only and without limitation to the combination of features necessary for carrying the invention into effect.

By physiological pH and temperature, it is meant the range in temperature and pH compatible with the digestive system within an animal, for example but not limited to, poultry and swine. For example, a suitable physiological temperature range is from about 35 to about 60°C, more preferably, this range is from about 40 to about 50°C. Similarly, a suitable physiological pH range is from about pH 3.0 to about 7.0, preferably, this range is from about pH 3.5 to about 6.0. The time required for the digestion of feed within the gut of an animal varies from animal to animal. For example, in swine digestion of feed is from about 2 to about 4 hours, while in poultry it is up to about 12 hours.

By high activity at physiological pH and temperature, it is meant that the enzyme exhibits at least 40% of its optimum activity at physiological pH and temperature. The optimum pH and temperature-range can be outside the physiological range, provided that the enzyme exhibits at least 40% of its optimum activity within the physiological range, for example from about 40 to about 50°C and pH from about 3.5 to about 6. Examples 4 and 5 describe the determination of a suitable xylanase enzyme that exhibits these properties.

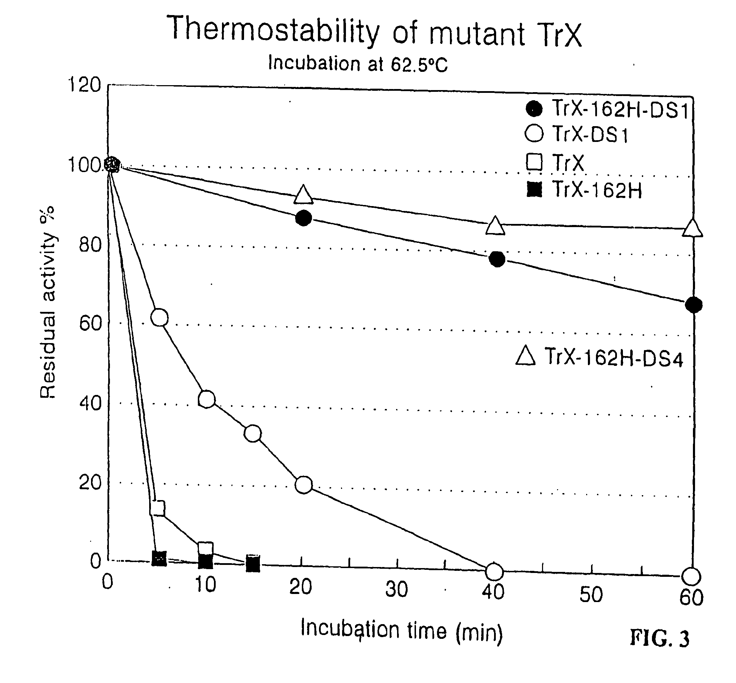

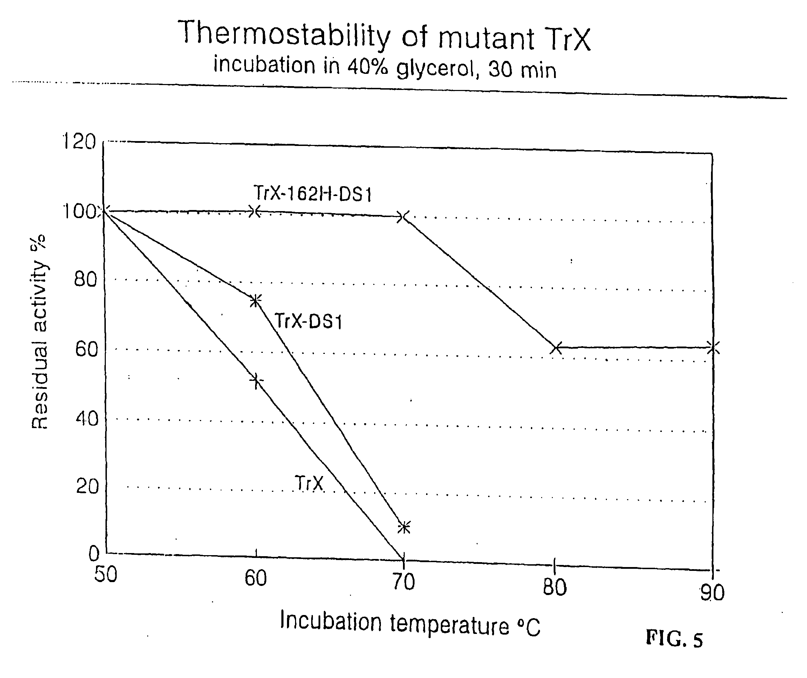

"Thermostable" or "thermostability" as used herein refer to a property of an enzyme. An enzyme is considered to be thermostable if it exhibits at least one of the following properties:
1) the enzyme exhibits at least 30% of its optimal activity following a pre-incubation step of 30 min at 70°C, 80°C, or 90°C, at pH 5.0, in the presence of a stabilizing agent such as 40% glycerol. Preferably, the enzyme exhibits at least 40% of its optimal activity following a 30 min, 70°C pre-incubation step in glycerol, for example but not limited to, TrX-162H-DS1 (Figure 5);
2) the enzyme exhibits 30% of its optimal activity following a pre-incubation step of 30 or 60 min at 62.5°C in the absence of a stabilizer. Preferably, the enzyme exhibits at least 40% of its optimal activity following a 30 min pre-incubation, for example but not limited to, TrX-162H-DS1 and TrX-162H-DS4 (Figure 3);
3) the enzyme exhibits at least 30% of its optimal activity following a preincubation step of 30 min at 64°C in the absence of a stabilizer. Preferably, the enzyme exhibits at least 40% of its optimal activity following the 30 min, 64°C pre-incubation step, for example but not limited to, TrX-162H-DS1 and TrX-162H-DS4 (Figure 4); or
4) the enzyme exhibits at least 30% of its optimal activity following a preincubation step of 30 min at 68°C in the absence of a stabilizer. Preferably, the enzyme exhibits at least 40% of its optimal activity following the 30 min, 68°C pre-incubation step, for example but not limited to, TrX-162H-DS1 and TrX-162H-DS4 (Figure 4). In each of the above cases, the optimal activity of the enzyme is determined at an optimal pH and temperature for that enzyme in the presence or absence of stabilizer as required.

By "TrX numbering" it is meant the numbering associated with the position of amino acids based on the amino acid sequence of TrX (Xyn II - Table 1; Tr2 - Figure 1). As disclosed below and as is evident upon review of Figure 1, Family 11 xylanases exhibit a substantial degree of sequence homology. Therefore, by aligning the amino acids to optimize the sequence similarity between xylanase enzymes and by using the amino acid numbering of TrX as the basis for numbering, the positions of amino acids within other xylanase enzymes can be determined relative to TrX.

By modified xylanase, it is meant the alteration of a xylanase molecule using techniques that are known to one of skill in the art. These techniques include, but are not limited to, site directed mutagenesis, cassette mutagenesis, synthetic oligonucleotide construction, cloning and other genetic engineering techniques. Alterations of a xylanase enzyme, in order to produce a modified xylanase may also arise as a result of applying techniques directed at inducing mutations within native or genetically engineered xylanases via the addition of known chemical mutagens, UV exposure, or other treatments known to induce mutagensis within a host organisms that express a xylanase of interest. Such techniques are well known within the art.

Table 1 lists the Family 11 xylanases free of cellulase activity. These enzymes share extensive amino acid sequence similarity and possess amino acids common to Family 11, for example two glutamic acid (E) residues serving as the essential catalytic residues, amino acids 86 and 177 (using TrX numbering). Structural comparisons of several Family 11 xylanases via X-ray crystallography indicates that these Family 11 xylanases of bacterial and fungal origins share the same general molecular structure (see for example US 5,405,769; Arase, A., Yomo, T., Urabe, I., Hata, Y., Katsube, Y. and Okada, H, 1993, FEBS Lett. 316:123-127). Most of the family 11 xylanases identified so far are mesophilic and have low-molecular mass (20kDa).

**TABLE 1: Family 11 xylanases**

| Microbe | Xylanase | Ref. in Figure 1 | Sequence Listing |
|---|---|---|---|
| *Aspergillus niger* | Xyn A | An | SEQ ID NO: I |
| *Aspergillus kawachii* | Xyn C | | |
| *Aspergillus tubigensis* | Xyn A | At | SEQ ID NO: 2 |
| *Bacillus circulans* | Xyn A | Bc | SEQ ID NO: 3 |
| *Bacillus pumilus* | Xyn A | Bp | SEQ ID NO: 4 |
| *Bacillus subtilis* | Xyn A | Bs | SEQ ID NO: 5 |
| *Cellulomonas fimi* | Xyn D | | |
| *Chainia spp.* | Xyn | | |
| *Clostridium acetobutylicum* | Xyn B | Ca | SEQ ID NO: 6 |
| *Clostridium stercorarium* | Xyn A | Cs | SEQ ID NO: 7 |
| *Fibrobacter succinognees* | Xyn C | | |
| *Neocallimasterix patriciarum* | Xyn A | | |
| *Nocardiopsis dassonvillei* | Xyn II | | |
| *Ruminococcus flavefaciens* | Xyn A | Rf | SEQ ID NO: 8 |
| *Schizophyllum commune* | Xyn | Sc | SEQ ID NO: 9 |
| *Streptomyces lividans* | Xyn B | SIB | SEQ ID NO: 10 |
| *Streptomyces lividans* | XynC | S1C | SEQ ID NO: 11 |
| *Streptomyces sp. No. 36a* | Xyn | Ss | SEQ ID NO: 12 |
| *Streptomyces thermoviolaceus* | XynII | | |
| *Thermomonospora fusca* | Xyn A | Tf | SEQ ID NO: 13 |
| *Trichoderma harzianum* | Xyn | Th | SEQ ID NO: 14 |
| *Trichoderma reesei* | Xyn I | Tr1 | SEQ ID NO: 15 |
| *Trichoderma reesei* | Xyn II | Tr2 | SEQ ID NO: 16 |
| *Trichoderma viride* | Xyn | Tv | SEQ ID NO: 17 |

It is considered within the scope of the present invention that xylanases, including Family 11 xylanases for example but not limited to *Trichoderma reesei* xylanase II, *Trichoderma reesei* xylanase I, *Trichoderma viride* xylanase, *Streptomyces lividans* xylanase B and *Streptomyces lividans* xylanase C, may be modified following the general approach and methodology as outlined herein.

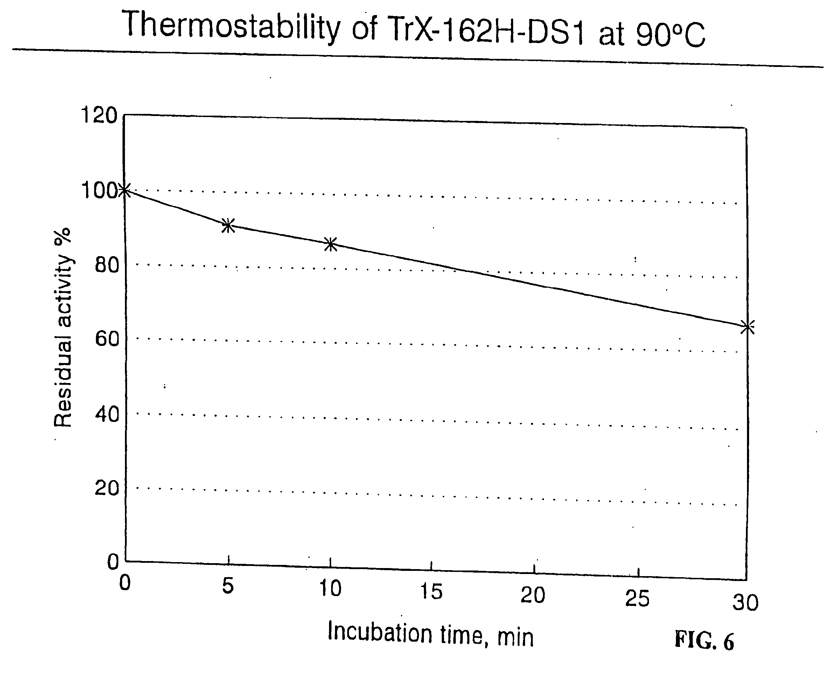

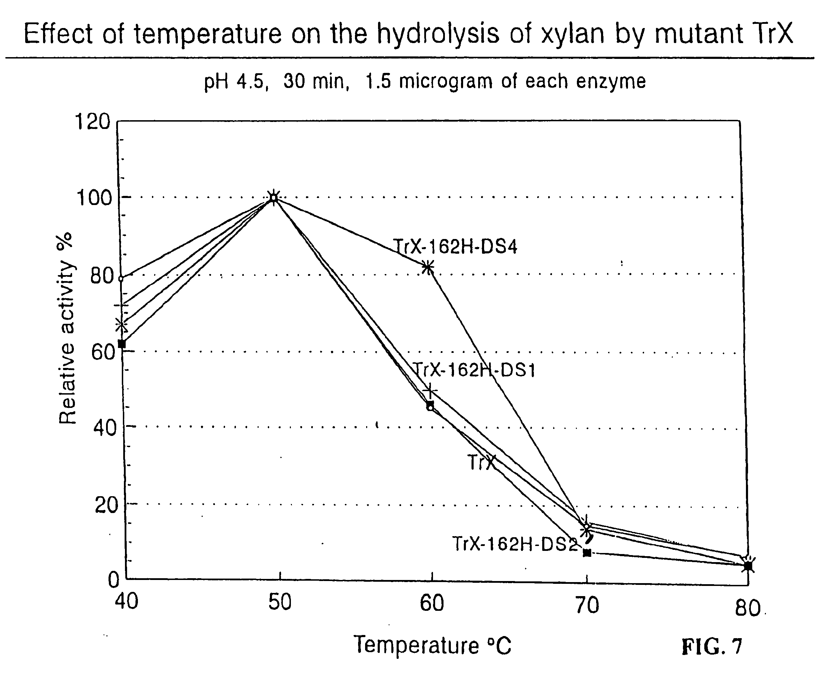

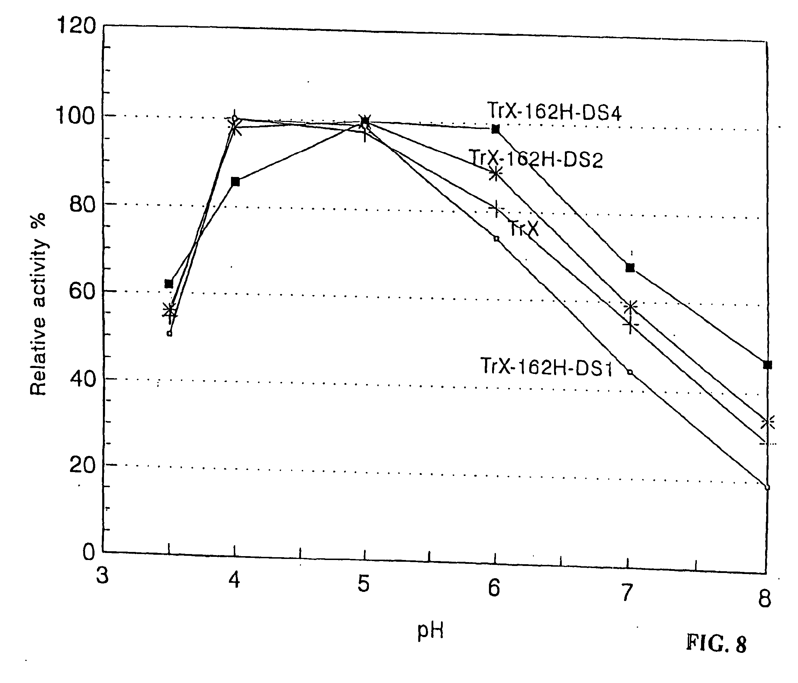

The present invention also relates to modified xylanase enzymes that exhibit increased thermostability while maintaining high activity at physiological pH and temperature. For example, and without wishing to limit the present invention in any manner, a modified *Trichoderma reesei* xylanase (TrX) is disclosed that exhibits increased thermostability while maintaining pH and temperature optima at or near physiological range. Two modifications in the TrX were combined in order to obtain a novel xylanase (TrX-162H-DS1). The first modification includes a double mutation to create two cysteines for the formation of a single disulfide bond. Such a modification has been described for *Bacillus circulans* xylanase (C100/C148; BcX amino acid numbering) in US 5,405,769. However, this mutation bestows only a minor increase in the ability of the enzyme to withstand high temperatures (see TrX-DS1, Figures 3-5) and this modification is not adequate to produce an enzyme capable of surviving high temperatures associated with the pelleting process. When this mutation is combined with a second mutation as per the teaching of this invention, involving the substitution of a basic amino acid such as histidine (H) for glutamine (Q) in position 162, the resultant combination mutant xylanase exhibits the desired properties of thermostability (TrX-162H-DS1; see Figures 5 and 6), and greater than 40% of optimum activity at physiological pH (Figure 8), and temperature (Figure 7).

Another mutant xylanase in the present invention, TrX-162H-DS4 differs from TrX-162H-DS1 by possessing an additional disulfide (108/158, that is between positions 108 and 158). This type of double disulfide mutant has previously been described for the xylanase of *Bacillus circulans* (C98/C152, 100/148; BcX amino acid numbering; Wakarchuck et al , 1994 Protein Engineering, 7:1379-1386). The BcX mutant does not comprise an equivalent basic amino acid (e.g. H for Q at position 162) substitution as disclosed herein. The mutant TrX-162H-DS4 shows a dramatic increase of thermostability (see Figure 4(a)), with an increase in the T₅₀ of TrX-162H-DS4 of 14°C. This is an improvement over the prior art double disulfide BcX mutant which exhibits an increase in the T₅₀ of 10°C, thereby demonstrating the contribution of the Q162H mutation in the disulfide mutants of TrX.

The present invention also pertains to additional mutations that have been found to be effective in producing a xylanase that exhibits thermostability and a desirable pH profile. An example of such mutations may be found in, but are not limited to, TrX-DS8. TrX-DS8 includes the mutations listed for N1-TX13 as disclosed in US 5,759,840, namely N1OH, Y27M and N29L, and also includes N44D, Q125A, I129E, Q162H and a disulfide bond between positions 110 and 154. Trx-DS8 exhibits the property of thermostability (Figure 4(b)), a pH profile parallelling that of TrX-162-DS1, and greater than 40 % of optimum activity at physiological pH, and temperature.

Xylanase enzymes comprising the substitution of H for Q at position 162 (termed Q162H) in isolation has been reported in US 5,759,840, however, these mutants exhibited no improvement in thermostability or other properties over natural TrX. However, by combining these two modifications, several novel xylanases (TrX-162H-DS1, TrX-162H-DS2 and TrX-162H-DS4) were obtained with improved thermostability. This property was not observed with either mutation alone. Furthermore, these modified xylanases exhibit high activity at or near physiological temperature and pH. These mutations are also found in Trx-DSB, which also exhibits improved thermostability and high activity at or near physiological conditions.

Following the methods of the present invention novel xylanase enzymes may be obtained that are far more suitable for feed pelleting applications than enzymes currently available. Similar modifications may be made in other Family 11 xylanases, including xylanase enzymes obtained from *Trichoderma, Streptomyces* and *Schizophyllum.*

In use, the formulation of the feed enzyme can improve the enzymes thermostability, as adsorption into feed improves stability as the enzyme is brought into contact with its substrate. Therefore, in determining thermostability of the xylanases of the present invention, xylanases were characterized in the presence and absence of stabilizing agents, for example but not limited to glycerol. Fisk and Simpson (1993) have reported that 40% glycerol enhanced the temperature tolerance of wild type TrX by less than + 10°C, however, this is much less stability than the enzymes of the present invention. The combination-mutant xylanases of the present invention can tolerate incubation in buffer at a higher temperature (59-69°C), as compared to natural xylanase (55°C; also see Figure 3 and 4). In the presence of 40% glycerol, the combination mutants can retain a substantial portion of their activity at 70 to 90°C (see figure 5), while the natural xylanase is totally inactivated at these temperatures..

One of the modifications to the combination mutant xylanase as proposed herein is the substitution of amino acid 162 (TrX numbering, based on Tr2 in Figure 1; which for TrX is glutamine) with the basic amino acid histidine (termed Q162H). However, it is considered within the scope of the present invention that other amino acids may also be substituted at this position. Preferably the substituted amino acid is basic (positively charged), for example lysine (Q162K) or arginine (Q162R). It has been observed herein that the substitution at the position 162, or its equivalent in other Family 11 xylanases, by a basic amino acid such as histidine can greatly improve the thermostability of a xylanase enzyme that comprises at least one intramolecular disulfide bond. Importantly, it has also been observed herein that this substitution at position 162 not only increases thermostability but also does not significantly change the temperature and pH profiles, and the specific activity of the modified xylanase.

Histidine-162 residue (TrX numbering) in the combination mutant is found in several natural Family 11 xylanases, such as those of *Trichoderma harzianum, Aspergillus niger, var. awamori, Aspergillus tubigensis, Thermomonospora fusca, Bacillus circulans* and *Bacillus subtilis* in the corresponding position. Similarly, *Clostridium acetobutylicum* comprises a lysine at this equivalent position. However, all, of these xylanases, with the exception of the *Thermomonospora fusca* xylanase, are produced by mesophilic hosts and exhibit low thermostability. As a result there is no evidence to suggest any beneficial effect on thermostability by presence of a basic amino acid residue at this position. In the *Thermomonospora fusca* xylanase, the N-terminal sequence (1-29) which is distant from the site of the present invention, has been shown to contribute to thermostability, and there is no evidence to suggest that thermostability may be associated with a histidine at this equivalent position (i.e. TrX 162).

This invention is also directed to Trichoderma, Streptomyces or Schizophyllum xylanases that comprise at least one modification that results in increased thermostability while maintaining high activity at physiological pH and temperature. For example, native *Schizophyllum commune* xylanase has a disulfide bond at positions 110/154 (TrX numbering). However, this enzyme exhibits low thermostability, Therefore, this enzyme can be modified using the methods of the present invention to substitute a basic amino acid, either histidine, arginine or lysine for the naturally occurring leucine at position 200 of *Schizophyllum commune* (which is equivalent to position 162 using TrX numbering; see Figure 1; Sc). Therefore, increased thermostability can be achieved through a one-step modification.

Also considered within the scope of the present invention are Trichoderma, Streptomyces or Schizophyllum combination mutants comprising both an intramolecular disulfide bond and a basic amino acid substitution as outlined above. The intramolecular disulfide bond may arise as a result of a mutation at one or more specific residues, for example (per TrX numbering):
- residues-110/-154, for example, but not limited to TrX-162H-DS1 or Trx-DS8;
- residues-108/-158, for example, but not limited to TrX-162H-DS2; or
- residues-108/-158, -110/-154, for example, but not limited to TrX-162H-DS4.

Also considered within the scope of the present invention are modifications of thermostable Trichoderma, Streptomyces or Schizophyllum xylanases, for example. These modifications maintain the thermostability of the native enzyme, yet alter the pH and temperature optima so that they exhibit high activity at physiological pH and temperature not normally associated with the enzyme.

**TABLE 2: Modified xylanases**

| **XYLANASE** | **DESCRIPTION** |
|---|---|
| wild type TrX | wild type *T. reesei* xylanase. |
| TrX-162H | TrX mutant with mutation Q162H. |
| TrX-DS1 | TrX mutant with an intramolecular disulfide bond between positions-110 and 154. |
| TrX-162H-DS1 | TrX mutant with two mutations, (i) a disulfide bond between posieions-110 and 154. and (ii) mutation Q162H. |
| TrX-162H-DS2 | TrX mutant with two mutations, (i) an intramolecular disulfide bond between positions-108 and 158, and (ii) mutation Q162H. |
| TrX-162H-DS4 | TrX mutant with two mutations. (i) two intramolecular disulfide bonds at residues-110/154 and residues-108/158. and (ii) mutation Q162H. |
| TrX-DS8 | Trx mutant with i) an intramolecular disulfide bond between positions-110 and 154, and ii) comprising mutations N10H, Y27M, N29L. N44D, Q125A, 1129E. and Q162H |

The present invention will be further illustrated in the following examples. However it is to be understood that these examples are for illustrative purposes only, and should not be used to limit the scope of the present invention in any manner.

### Examples:

### Example 1: Construction of the Trichoderma reesei mutant xylanases

Basic recombinant DNA methods like plasmid preparation, restriction enzyme digestion, polymerase chain reaction, oligonucleotide phosphorylation, ligation, transformation and DNA hybridization were performed according to well-established protocols familiar to those skilled in the art (Sung, W. L., Yao, F.-L., Zahab, D. M. and Narang, S. A. (1986) Proc. Natl. Acad. Sci. USA 83:561-565) or as recommended by the manufacturer of the enzymes or kit. The buffer for many enzymes have been supplied as part of a kit or constituted following to the instruction of the manufacturers. Restriction enzymes, T4 polynucleotide kinase and T4 DNA ligase were purchased from New England BioLabs LTD, Mississauga, Ont. A precursor plasmid pXYbc has previously prepared and published (Sung, W. L., Luk, C. K., Zahab, D. M. and Wakarchuk, W. (1993) Protein Expression Purif. 4:200-206; US 5,405,769). A commonly used E. coli strain, HB101 (clonetech Lab, Palo Alto, CA) was used as transformation and expression host for all gene construct. Birchwood xylan was purchased from Sigma (St. Louis, Mo). Hydroxybenzoic acid hydrazide (HBAH) was purchased from Aldricht. Oligonucleotides were prepared with an Applied Biosystem DNA synthesizer, model 380B. Xylanase assays have been performed in a covered circulating water bath (Haake type F 4391) with a fluctuation of "0.1°C. Temperature of the water bath was confirmed with a thermocouple.

### A. Construction of the precursor plasmid pTrX

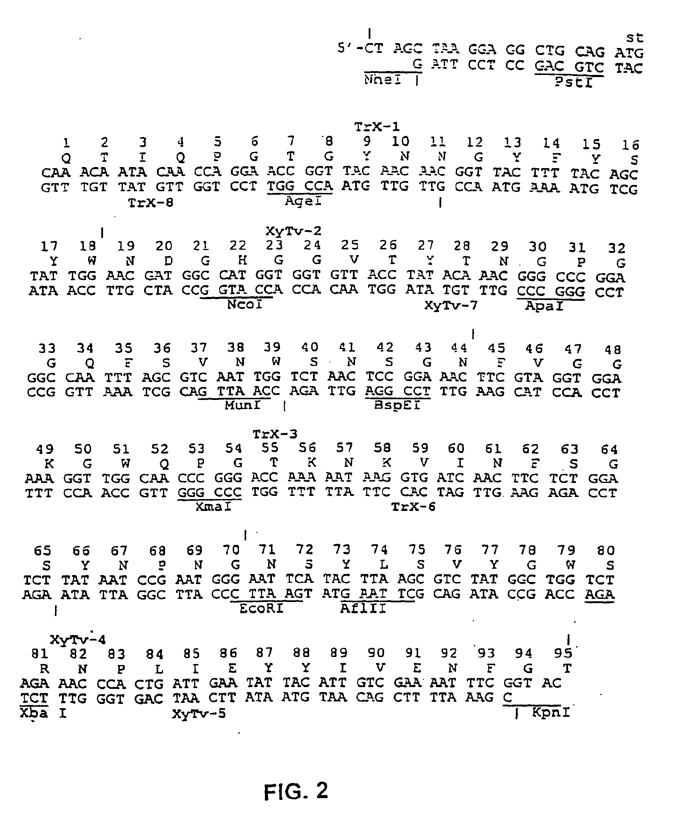

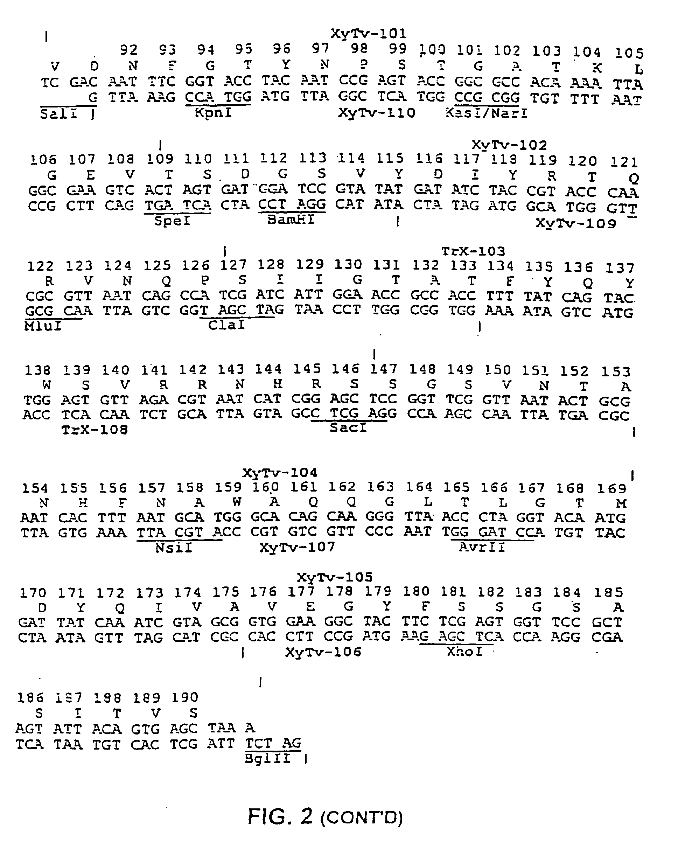

The precursor plasmid pTrX for all subsequent mutations is published (Sung et al, 1995). This plasmid is derived from a pUC119 plasmid with a synthetic nucleotide sequence encoding a *Trichoderma reesei* xylanase inserted (Figure 2). Expression of this xylanase and other mutant xylanases subsequently described are under the control of the lac promoter of the pUC plasmid. The total assembly of the gene required two stages, initially for the (92-190) region, then followed by the (1-92) region. The protocol for the construction of this gene is routine and identical to the standard published procedure for many other genes. It required enzymatic phosphorylation of overlapping synthetic oligonucleotides which encodes xylanase. This was followed by their ligation into a appropriately cut plasmid pUC119.

Initially ten overlapping oligonucleotides:

| | |
|---|---|
| XyTv-101, | SEQ ID NO:28 |
| XyTv-102, | SEQ ID NO:29 |
| TrX-103, | SEQ ID NO:30 |
| XyTv-104, | SEQ ID NO:31 |
| XyTv-105, | SEQ ID NO:32 |
| XyTv-106, | SEQ ID NO:33 |
| XyTv-107, | SEQ ID NO:34 |
| TrX-108, | SEQ ID NO:35 |
| XyTv-109, | SEQ ID NO:22 |
| XyTv-110, | SEQ ID NO:36 |

encoding the TrX(92-190) sequence (Figure 2), were designed with codon usage frequency imitating that of *E. coli* (Chen et al. 1982). The SalI and BglII cohesive ends of two terminal oligonucleotides enabled the enzymatic ligation of the ten fragments to the linearized plasmid pXYbc. The ten oligonucleotides (50 pmol, 1 L for each) encoding the TrX(92-190) was phosphorylated in a mixture containing 10X standard kinase buffer (0.4 L), 1mM ATP (4 L), T4 DNA kinase (5 units), and water (3 L). Phosphorylation reaction was carried out for 1 h at 37°C. The solutions were then combined and heated to 70°C for 10 min. After being cooled slowly to room temperature, the combined solutions were added to a mixture of 4mM ATP (3.5 L), EcoR1-HindIII linearized plasmid pUC119 (0.1 pmol), and T4 DNA ligase (3.5 L) and incubated at 12°C for 20 h. Aliquots of the ligation mixture were used to transform *E*. *coli* HB101 in YT plate (8 g yeast extract, 5 g bacto-tryptone, 5 g NaCl, 15 g of agar in 1 L of water) containing ampicillin (100 mg/L).

For the preparation of a hybridization probe, one of the oligonucleotide XyTv-110 (10 pmol, 1 L) was phosphorylated ³²P-ATP (10 pmol, 3 L) in T4 DNA kinase (1 L), 10X kinase buffer (1 L), and water (4 L) at 37°C for 1 h.

Transformants were selected randomly for hybridization analysis. Colonies were grown on nylon filters on YT plates with ampicillin overnight. They were then denatured with 0.5N NaOH -1.5M NaCl (10 min) and neutralized with 0.5N Tris-HCl (pH 7.0) - 1.5M NaCl (10 min). After irradiation by UV of 254 nm for 8 min, the filters were washed with 6X SSC - 0.05% Triton X-100 for 30 min. Cell debris was scraped off completely. After another 30 min. in fresh solution, the duplicate filters were transferred individually into separate mixtures of 6X SSC - 1 % dextran sulphate - 0.05 % TritonX-100 - 1X Denhardt's hybridization fluid. The ³²P-labelled probe was added to the filter. After 16 h at 45°C, the filter was washed twice with 6X SSC - 0.05 % TritonX-100 at room temperature for 5 min. and then at 65°C for 30 min. Positively hybridized clones with the intermediate plasmid pBcX.TrX were identified by auto-radiographic analysis.

The above protocol, involving enzymatic phosphorylation of synthetic overlapping oligonucleotides and ligation into a linearized plasmid, has again been used in the assembly of the TrX(1-92) region and in the cassette mutagenesis for the subsequent generation of other mutant series described in this invention.

For the assembly of the TrX(1-92) region to complete the full-length *Trichoderma* gene, the intermediate plasmid pBcX.TrX was linearized by NheI and KpnI endonucleases to release the DNA insert for BcX(l-83). With Nhel and Kpnl cohesive ends, eight overlapping oligonucleotides:

| | |
|---|---|
| TrX-1, | SEQ ID NO:37 |
| XyTv-2, | SEQ ID NO:38 |
| TrX-3, | SEQ ID NO:39 |
| XyTv-4, | SEQ ID NO:40 |
| XyTv-5, | SEQ ID NO:41 |
| TrX-6, | SEQ ID NO:42 |
| XyTv-7, | SEQ ID NO:43 |
| TrX-8, | SEQ ID NO:44, |

encoding the published TrX(1-91) sequence were ligated into the linearized plasmid pBcX.TrX (Figure 2), via the protocol described above. The new plasmid pTrX therefore harbored a synthetic TrX gene (SEQ ID NO: 18).

All mutant xylanases described below have been constructed via the method of cassette mutagenesis as described above. The protocol for the cassette mutagenesis was identical to that for gene assembly fully described above. Such cassette mutagenesis involved (i) enzymatic phosphorylation of overlapping synthetic oligonucleotides, (ii) their ligation with the linearized plasmid, (iii) transformation into the *E. coli* HB101 competent cells, (iv) identification of the mutant transformants via hybridization with the labelled oligonucleotide as probe, and (v) confirmation of the mutation through dideoxy nucleotide sequencing.

### B. Construction of the plasmid pTrX-DS1

The mutant TrX-DS1 (SEQ ID NO's:54, 55) was identical to TrX with a covalent disulfide bond between residues-110 and 154. This was accomplished through two single mutations, ie. conversion of both residues serine-110 and asparagine-154 to cysteine. Upon expression of the mutant xylanase, these two cysteine residues will form a disulfide bond. The construction of the plasmid pTrX-DS1 was through ligation of the following overlapping phosphorylated oligonucleotides:

| | |
|---|---|
| TX-110C | SEQ ID NO: 19, |
| TX-110C-2 | SEQ ID NO:20, |
| TX-103b | SEQ ID NO:21, |
| XyTv-109 | SEQ ID NO:22, |
| TX-108b | SEQ ID NO:23, |
| TX-154C | SEQ ID NO:24, |
| TX-154C-2 | SEQ ID NO:25, |

into KasI/AvrII-linearized plasmid pTrX in a cassette mutagenesis as shown below.

### C. Construction of the plasmid pTrX-162H-DS1

The mutant TrX-162H-DS 1 (SEQ ID NO:56) was identical to TrX-DS 1 with a single mutation of glutamine-162 into histidine. The construction of the plasmid pTrX-162D-DS 1 was through ligation of oligonucleotides:

| | |
|---|---|
| TX-162H-3 | SEQ ID NO: 26, and |
| TX-162H-4 | SEQ ID NO: 27 |

into SphI/AvrII-linearized plasmid pTrX-DS1 in a cassette mutagenesis, as shown below.

### D. Construction of the plasmid pTrX-162H-DS2

The mutant TrX-162H-DS2 (SEQ ID NO's:57,58) was identical to TrX, but with a covalent disulfide bond between residues-108 and -158, and a mutation glutamine-162 to histidine. The 108/110 disulfide required two single mutations, ie. conversion of both residues valine-108 and alanine-158 to cysteine. Upon expression of the mutant xylanase, these two cysteine residues will form a disulfide bond. The construction of the plasmid pTrX-162H-DS2 was through ligation of the following overlapping phosphorylated oligonucleotides:

| | |
|---|---|
| TX-108C | SEQ ID NO:45, |
| TX-108C-2 | SEQ ID NO:46, |
| TX-103b | SEQ ID NO:21, |
| XyTv-109 | SEQ ID NO:22, |
| TX-108b | SEQ ID NO:23, |
| TX-158C-162H | SEQ ID NO:47, and |
| TX-158C-162H-2 | SEQ ID NO:48 |

into the KasI/AvrII-linearized plasmid pTrX in a cassette mutagenesis as shown below.

### E. Construction of the plasmid pTrX-162H-DS4

The mutant TrX-162H-DS4 (SEQ IDNO's:59, 60) was identical to TrX, but with two covalent disulfide bonds 108/158 and 110/154 and a mutation glutamine-162 to histidine. The two disulfides required four single mutations, ie. conversion of the residues valine-108. serine-110, asparagine-154 and alanine-158 to cysteine. Upon expression of the mutant xylanase, these four cysteine residues will form two disulfide bonds. The construction of the plasmid pTrX-162H-DS4 was through ligation of the following overlapping phosphorylated oligonucleotides:

| | |
|---|---|
| TX-108C-110C | SEQ ID NO:49, |
| TX-108C-110C-2 | SEQ ID NO:50, |
| TX-103b | SEQ ID NO:21, |
| XyTv-109 | SEQ ID NO:22, |
| TX-108b | SEQ ID NO:23, |
| TX-154C-158C-162H | SEQ ID NO:51 and |
| TX-154C-158C-162H-2 | SEQ ID NO:52 |

into the Kasl/AvrII-linearized plasmid pTrX in a cassette mutagenesis, as shown below.

### F. Construction of TrX-DS8

The mutant TrX-DS8 was prepared using analogous methods as those outlined above in Sections A to E for the preparation of modified xylanases. TrX-DS8 incorporates the mutations found in N1-TX13 as disclosed in US 5,759,840. This mutations are N10H, Y27M and N29L. In addition, TrX-DS8 includes the following mutations: N44D, Q125A, I129E, Q162H and a disulfide bond between positions 110 and 154. The construction of the plasmid pTrX-DS8 was through ligation of overlapping phosphorylated oligonucleotides as described above.

Trx-DS8 exhibits the property of thermostability (Figure 4a), a pH profile parallelling that of TrX-162-DS1, and greater than 40% of optimum activity at physiological pH, and temperature.

### Example 2: Characterization of mutant xylanases

### A. Production of xylanases

The culture condition was identical to the well-established protocol described for other *E. coli*-expressed xylanases. A 5 ml of overnight inoculant in 2YT medium (16 g yeast extract, 10 g bacto-tryptone, 5 g NaCl, 1 L of water) containing ampicillin (100 mg/L) was added to 2YT medium (1 L) with ampicillin. The cultures were grown with shaking (200 rpm) at 37°C. After 16 hr, cells were harvested.

### B. Purification of different disulfide bond-containing mutant xylanases

Protein samples were prepared from cells by first making an extract of the cells by grinding 10 g of the cell paste with 25 g of alumina powder. After grinding to smooth mixture, small amounts (5 mL) of ice cold buffer A (10mM sodium acetate, pH 5.5 for BcX mutants) or buffer B (10mM sodium acetate, pH 4.6 for TX mutants) were added and the mixture ground vigorously between additions. The alumina and cell debris were removed by centrifugation of the mixture at 8000 x g for 30 min.

The crude extract was heated at 60°C for 15 min and centrifugation to remove a large amount of precipitate. The supernatant was acidified to pH 4.6, frozen at -20°C overnight, thawed and centrifuged to remove more precipitate.

After the above pretreatment, the cell extract committed to column chromatography and was pumped onto a 50 mL bed volume, S-Sepharose fast flow, cation exchange column (Kabi-Pharmacia, Canada), equilibrated in buffer A. The xylanase was eluted with a 300 mL linear gradient of 0 to 0.3M NaCl in buffer A at a flow rate of 3 mL/min. The xylanase elutes at 100 to 150 mL of the gradient. The fractions are checked on SDS-PAGE, and those fractions having most of the xylanase were pooled, and concentrated by ultrafiltration using 3000 dalton molecular weight cutoff membranes (Amicon YM3). The concentrated material (5 mL) was then applied to a 1.5 cm x 85 cm TSK-HW50S gel filtration column, equilibrated in 50 mM ammonium acetate pH 6. The xylanase eluted at a volume of 90 to 100 mL. These fractions were analyzed by SDS-PAGE, and the peaks pooled as pure xylanase. The protein was quantified using the extinction co-efficient at 280 nm.

### C. Standard assay for the measurement of enzymatic activity

The quantitative assay determined the number of reducing sugar ends generated from soluble xylan. The substrate for this assay was the fraction of birchwood xylan which dissolved in water from a 5% suspension of birchwood xylan (Sigma Chemical Co.). After removing the insoluble fraction, the supernatant was freeze dried and stored in a desiccator. The measurement of specific activity was performed as follows. Reaction mixtures containing 100 L of 30 mg/mL xylan previously diluted in assay buffer (50 mM sodium citrate, pH 5.5 or the pH optimum of the tested xylanase), 150 L assay buffer, 50 L of enzyme diluted in assay buffer were incubated at 40°C. At various time intervals 50 L portions were removed and the reaction stopped by diluting in 1 mL of 5mM NaOH. The amount of reducing sugars was determined with the hydroxybenzoic acid hydrazide reagent (HBAH) (Lever, 1972, Analytical Biochem 47:273-279). A unit of enzyme activity was defined as that amount generating 1 mol reducing sugar in 1 minute at 40°C.

For the comparison between mutant and the wild type xylanases (TABLE 3), the specific activities of a xylanase was converted to the relative activity which is its calculated in percentage as compared to the specific activity of the natural xylanase.

**TABLE 3. Relative activity of TrX xylanases**

| Xylanase | Relative activity % |
|---|---|
| natl. TrX | 100* |
| TrX | 103 |
| TrX-DS1 | 116 |
| TrX-162H-DS1 | 102 |
| TrX-162H-DS4 | 91 |

| | |
|---|---|
| * The specific activity of the natural TrX (770 U/mg) was normalized to 100%. | |

As can be seen form Table 3, the specific enzymatic activities of the mutant xylanases at 40°C have not been changed significantly as compared to the natural xylanases.

### Example 3: Thermostability of mutant xylanases

This was a test of the tolerance of xylanase to incubation at a set temperature, without any substrate. The xylanase (150 g/mL) in assay buffer (50 mM sodium citrate) was incubated at a set temperature or set period of time. Aliquots were cooled to room temperature (around 20°C), the residual enzymatic activity of all samples was determined via the HBAH assay at 40°C, as stated in Example 2C.

### (A) Effect of length of incubation

The effect of the length of incubation on the activity of xylanase samples was determined at 62.5°C at pH 5.5 (Figure 3). Aliquots were removed at 0, 5,10,20,30,40 and 60 min for the determination of residual activity. The residual enzymatic activity at 0 min was normalized to 100%.

After 5 mins of incubation, the wild type TrX and the Q162H mutant TrX-162H (US 5,759,840) almost lost all residual activity, while the mutant TrX-DS 1 with a disulfide bond, retained 60% of it residual activity. However, it retained only 20% of its activity at 20 mins and lost all activity at 40 min. In contrast, the mutant TrX-162H-DS1, with the additional mutation of Q162H, showed superior thermostability by retaining about 87% of its activity at 20 min, 78% at 40 min and 68% at 60 min. The mutant TrX-162H-DS4 with both 108/158 and 110/154 disulfide bonds retained 84% activity after 60 min..

### (B) Effect of incubation temperatures on the residual activity of mutant TrX.

Thermostability of mutant TrX enzymes was also determined by tolerance of different incubation temperatures. Samples of xylanases were incubated in 50 mM sodium citrate buffer (pH 5.5) at different temperatures (48, 52, 56; 60, 64, 68, 70 and 72°C) for 30 min. The residual enzymatic activity of the samples was determined, with the residual activity at 48°C normalized to 100% (see Figures 4(a) and 4(b)). The T₅₀, which is the incubation temperature allowing the maintenance of 50% residual activity after 30 min, was determined for each mutant TrX.

Without wishing to be bound by theory, the higher T₅₀ of TrX-162H-DS 1 (65°C) versus TrX-DS 1 (61 °C) demonstrates the enhancement of thermostability by the mutation Q162H in the disulfide mutants. The double disulfide mutant TrX-162H-DS4 also exhibited high stability with a T₅₀ gain of+14°C over the natural TrX. Comparison of T₅₀ of TrX-162H-DS1 (65°C) and TrX-162H-DS2 (59°C) indicates that the 110/154 disulfide in TrX-162H-DS 1 contributes greater thermostability than the 108/158 dislufide in the latter. TrX-DS8 also exhibited high thermostability, with a T₅₀ gain of +16 °C when compared to natural TrX.

### (C) Effective incubation temperature

In the following example, a model study of the effect of the enzyme formulation on thermostability of the combination mutant was conducted in the presence of an additive, glycerol. The unmodified TrX and the mutant TrX xylanases were incubated for 30 min at 20, 50, 60, 70, 80 and 90°C in a buffer (pH 5.0) with 40% glycerol. The residual activity was determined by the HBAH assay. The residual enzymatic activity at 0 min was normalized to 100% (Figure 5).

At 50°C, all TrX samples retained their enzymatic activity. At 60°C, the wild type TrX retained 75% of its activity while TrX-DS1 and TrX-162H-DS1 retained 80 and 100% respectively (Figure 5). At 70°C, TrX-DS1 and TrX-162H-DS1 maintained 10 and 98% respectively. At 90 min, the latter retained 65% of the residual activity.

### (D) Effect of incubation time on the residual activity of TrX-162H-DS1 at 90°C

Sample of TrX-162H-DS1 in 40% glycerol and buffer were incubated in a covered circulating water bath (Haake type F 4391, with a fluctuation of 0.1°C) at 90°C. Temperature of the water bath was confirmed with a thermocouple. Aliqiots were removed at 0, 5, 10 and 3 0 min for assay of residual activity. The residual enzymatic activity at 0 min was normalized to 100%.

At 5,10 and 30 min, TrX-162H-DS1 retained 90, 85 and 65% of the residual activity respectively (Figure 6).

### Example 4: Temperature/activity profile of mutant xylanases

This was a test on the effect on different temperatures to the enzymatic activity of the xylanase in the hydrolysis of soluble xylan. The procedure was identical to the standard assay (Example 2 C) with changes in the incubation temperature and time. The enzymes (1.5 µg/mL) and soluble xylanase in 50 mM sodium citrate buffer of pH 4.5 were mixed and incubated in a circulating water bath at different temperatures. After 30 min, the amount of reducing sugars released from xylan was determined by HBAH and was calculated as relative activity, with the value at temperature optimum as 100%.

The effect of temperature on the hydrolysis of xylan was shown in Figure 7. The natural TrX, TrX-DS1, TrX-162H-DS1, TrX-162H-DS2 and TrX-162H-DS4 enzymes all had the same temperature/activity profile, and the only difference is in the greater activity (80%) in mutant TrX-162H-DS4 as compared to the others (45%) at 60°C. These results indicate that the disulfide mutation, along with the Q162H mutation, has little or no effect on the optimal temperature (50°C) of TrX. In addition, all of the enzymes shown in the figure exhibit at least 40% of their optimum activity from about 40 to about 50 °C, which is suitable for feed pelleting applications.

### Example 5: pH/activity profile of mutant xylanases

This was a test of the effect of different pH on the enzymatic activity of the xylanase in the hydrolysis of soluble xylan at the approximate physiological temperature of digesta.

The procedure was identical to the standard assay (Example 2 C) with changes in the incubation temperature and time. The *Trichoderma* enzymes natural TrX and mutant TrX (3 0 µg/mL) and soluble xylan in 50 mM sodium citrate buffers of pH 3-8 were incubated together at 40°C for 7 min. The amount of reducing sugars released from xylan was determined by HBAH and was calculated as relative activity, with the value at pH optimum as 100%.

The profile of the effect of pH on the enzymatic activity of TrX, TrX-162H-DS 1 and TrX-162H-DS2 (Figure 8) are similar, thus indicating little or no effect of the mutations (disulfide bond formation and Q 162H) on the pH optimum. The pH profile for TrX-DS8 was also similar to these modified xylanases (data not shown). All of the enzymes shown in the figure exhibit at least 40% of their optimum activity from about pH 3.5 to about pH 6, which is suitable for feed pelleting applications.

The double disulfide mutant TrX-162H-DS4 differed by showing slightly greater activity at the pH range higher than 6. At the acidic pH of 4-6 TrX, TrX-162H-DS1, TrX-162H-DS2 and TrX-162H-DS4 maintained at least 75% optimal activity.

### References

Arase. A., Yomo, T., Urabe, I., Hata, Y., Katsube, Y. and Okada, H. (1993) FEBS Lett. 316:123-127.
Beauchemin, K.A., Jones, S.D.M., Rode, L.M., and Sewalt, V.J.H. (1997) Can. L. Animal Sci. 77:645-653.
Beauchemin, K.A. and Rode, L.M. (1997) in Dairy Research Results from the Agriculture and Agri-Food Canada Research Center, Lethbridge, 1E1:1-2.
Bedford, M.R and Classen, H.L. (1992) in Xylans and Xylanases, edited by J. Visser, G. Beldman, M. A. Kusters-van Someren and A. G. J. Voragen, published by Elsevier, Amsterdam, 1992. p361-370.
Cowan, D.A. (1995) Essays Biochem. 29:193-207.
Fisk, R. C. and Simpson, C. (1993) in Stability and Stabilization of Enzymes, edited by W. J. J. van den Tweel, A. Harder and R. M. Buitelaar, published by Elsevier Science Publishers B. V. pp323-328.
Gupta, M.N. (1991) Biotech. Applied Biochem. 14:1-11.
Irwin, D., Jung, E. D. and Wilson, D. B. (1994) Appl. Environ. Microbiol. 60:763-770.
Paloheimo, M., Mantyla, A., Vehmaanpera, J., Hakola, S., Lantto, R., Lahtinen, T., Parkkinen, E., Fagerstrom, R. and Suominen, P. (1997) in Carbohydrases from Trichoderma reesei and Other Microorganisms p255-264.
Sung, W. L.. Yao. F.-L., Zahab, D. M. and Narang, S. A. (1986) Proc. Natl. Acad. Sci. USA 83:561-565.
Sung, W. L., Luk, C. K., Zahab, D. M. and Wakarchuk, W. (1993) Protein Expression Purif. 4:200-206.
Sung, W. L., Luk, C. K., Chan, B., Wakarchuk, W., Yaguchi, M., Campbell, R, Willick, G., Ishikawa, K. and Zabab, D. M. (1995) Biochem. Cell. Biol. 73:253-259.
Torronen, A. and Rouvinen, J. (1995) Biochemistry 34:847-856.
Viveros, A., Brenes, A., Pizarro, M. and Castano, M. (1994) Animal Feed Sci. Technol. 48:237-251.
Wakarchuck W. W., Sung, W. L., Campbell, R L., Cunningham, A., Watson, D. C. and Yaguchi, M. (1994) Protein Engineering 7:1379-1386.

## Claims

1. An isolated modified Family 11 Trichoderma, Streptomyces or Schizophyllum, xylanase comprising at least one intramolecular disulphide bond, and a substituted basic amino acid at position 162 (*Trichodernia reesei* xylanase II numbering) or its equivalent, said position determined from sequence alignment of said isolated modified xylanase with said *Trichoderma reesei* xylanase II amino acid sequence defined in SEQ ID NO:16, said isolated modified xylanase exhibiting at least 40% of optimal activity from pH 3.5 to pH 6.0, and from 40 to 60°C, and at least 30% of optimal activity after:
a pre-incubation step for 30 minutes at 70°C, 80°C or 90°C in the presence of 40% glycerol;
a pre-incubation step for 30 or 60 minutes at 62.5°C in the absence of a stabilizer: or
a pre-incubation step of 30 minutes at 64°C or 68°C in the absence of a stabilizer.

2. The isolated modified xylanase of claim 1, wherein said basic amino acid is selected from the group consisting of lysine, arginine and histidine.

3. The isolated modified xylanase of claim 2, wherein said basic amino acid is histidine.

4. The isolated modified xylanase of claim 1 comprising two disulfide bridges.

5. The isolated modified xylanase of claim 1 selected from the group consisting of TrX-162H-DS 1, TrX-162H-DS2, TrX-162H-DS4, and TrX-DS8.

6. The isolated modified xylanase of claim 5, wherein said xylanase is TrX-162H-DS1.

7. The isolated modified xylanase of claim 5, wherein said xylanase is TrX-162H-DS2.

8. The isolated modified xylanase of claim 5, wherein said xylanase is TrX-162H-DS4.

9. The isolated modified xylanase of claim 5, wherein said xylanase is TrX-DS8.

10. A method of preparing animal feed comprising applying the isolated modified xylanase of claim 1 onto said animal feed to produce a xylanase-animal feed combination, and heat sterilizing said xylanase-animal feed combination.

11. The method of claim 10, wherein said animal feed is a poultry or swine feed.

12. The isolated modified xylanase of claim 1 comprising one disulfide bridge.

13. The isolated modified xylanase of claim 1, said xylanase obtained from an organism selected from the group consisting of *Schizophyllum commune, Streptomyces lividans, Trichoderma reesei,* and *Trichoderma viride.*

## Patentansprüche

1. Isolierte modifizierte *Trichoderma, Streptomyces oder Schizophyllum* Xylanase der Familie 11 mit wenigstens einer intramolekularen Disulphidbindung und einer substituierten basischen Aminosäure an Position 162 (*Trichoderma reesei* Xylanase II Nummerierung) oder deren Äquivalent, wobei die genannte Position anhand eines Sequenzalignments der genannten isolierten modifizierten Xylanase mit der genannten, in SEQ ID Nr. 16 definierten *Trichoderma reesei* Xylanase II Aminosäuresequenz bestimmt wird, wobei die genannte isolierte modifizierte Xylanase wenigstens 40 % optimale Aktivität von pH 3,5 bis pH 6,0 und von 40 bis 60 °C und wenigstens 30 % optimale Aktivität nach Folgendem aufweist:
einem 30-minütigen Vorinkubationsschritt bei 70 °C, 80 °C oder 90 °C in Gegenwart von 40 % Glycerol;
einem 30- oder 60-minütigen Vorinkubationsschritt bei 62,5 °C in Abwesenheit eines Stabilisators; oder
einem 30-minütigen Vorinkubationsschritt bei 64 °C oder 68 °C in Abwesenheit eines Stabilisators.

2. Isolierte modifizierte Xylanase nach Anspruch 1, wobei die genannte basische Aminosäure aus der Gruppe bestehend aus Lysin, Arginin und Histidin ausgewählt ist.

3. Isolierte modifizierte Xylanase nach Anspruch 2, wobei die genannte basische Aminosäure Histidin ist.

4. Isolierte modifizierte Xylanase nach Anspruch 1, die zwei Disulfidbrücken umfasst.

5. Isolierte modifizierte Xylanase nach Anspruch 1, ausgewählt aus der Gruppe bestehend aus TrX-162H-DS1, TrX-162H-DS2, TrX-162H-DS4 und TrX-DS8.

6. Isolierte modifizierte Xylanase nach Anspruch 5, wobei die genannte Xylanase TrX-162H-DS1 ist.

7. Isolierte modifizierte Xylanase nach Anspruch 5, wobei die genannte Xylanase TrX-162H-DS2 ist.

8. Isolierte modifizierte Xylanase nach Anspruch 5, wobei die genannte Xylanase TrX-162H-DS4 ist.

9. Isolierte modifizierte Xylanase nach Anspruch 5, wobei die genannte Xylanase TrX-DS8 ist.

10. Verfahren zur Herstellung von Tierfutter, das das Aufbringen der isolierten modifizierten Xylanase nach Anspruch 1 auf das genannte Tierfutter zum Erzeugen einer Xylanase-Tierfutter-Kombination und das Hitzesterilisieren der genannten Xylanase-Tierfutter-Kombination beinhaltet.

11. Verfahren nach Anspruch 10, wobei das genannte Tierfutter Geflügel- oder Schweinefutter ist.

12. Isolierte modifizierte Xylanase nach Anspruch 1, die eine Disulfidbrücke umfasst.

13. Isolierte modifizierte Xylanase nach Anspruch 1, wobei die genannte Xylanase von einem Organismus erhalten wird, der aus der Gruppe bestehend aus *Schizophyllum commune, Streptomyces lividans, Trichoderma reesei* und *Trichoderma viride* ausgewählt ist.

## Revendications

1. Une xylanase de la famille 11 de *Trichoderma, Streptomyces ou Schizophyllum* modifiée isolée comprenant au moins une liaison disulfure intramoléculaire, et un acide aminé basique substitué à la position 162 (numérotation de la xylanase II de *Trichoderma reesei*) ou son équivalent, ladite position déterminée à partir de l'alignement de séquence de ladite xylanase modifiée isolée avec ladite séquence d'acides aminés de la xylanase II de *Trichoderma reesei* définie en SEQ ID NO:16, ladite xylanase modifiée isolée présentant au moins 40% d'activité optimale de pH 3,5 à pH 6,0, et de 40 à 60°C, et au moins 30% d'activité optimale après :
une phase de pré-incubation de 30 minutes à 70°C, 80°C ou 90°C en présence de glycérol à 40%;
une phase de pré-incubation de 30 ou 60 minutes à 62,5°C en l'absence de stabilisateur; ou
une phase de pré-incubation de 30 minutes à 64°C ou 68°C en l'absence de stabilisateur.

2. La xylanase modifiée isolée de la revendication 1, dans laquelle ledit acide aminé basique est sélectionné à partir du groupe constitué de la lysine, l'arginine et l'histidine.

3. La xylanase modifiée isolée de la revendication 2, dans laquelle ledit acide aminé basique est l'histidine.

4. La xylanase modifiée isolée de la revendication 1 comprenant deux ponts disulfures.

5. La xylanase modifiée isolée de la revendication 1 sélectionnée à partir du groupe constitué de TrX-162H-DS1, TrX-162H-DS2, TrX-162H-DS4, et TrX-DS8.

6. La xylanase modifiée isolée de la revendication 5, dans laquelle ladite xylanase est TrX-162H-DS1.

7. La xylanase modifiée isolée de la revendication 5, dans laquelle ladite xylanase est TrX-162H-DS2.

8. La xylanase modifiée isolée de la revendication 5, dans laquelle ladite xylanase est TrX-162H-DS4.

9. La xylanase modifiée isolée de la revendication 5, dans laquelle ladite xylanase est TrX-DS8.

10. Une méthode de préparation d'alimentation animale comprenant l'application de la xylanase modifiée isolée de la revendication 1 sur ladite alimentation animale afin de produire une combinaison xylanase-alimentation animale, et la stérilisation thermique de ladite combinaison xylanase-alimentation animale.

11. La méthode de la revendication 10, dans laquelle ladite alimentation animale est une alimentation pour la volaille ou les porcs.

12. La xylanase modifiée isolée de la revendication 1 comprenant un pont disulfure.

13. La xylanase modifiée isolée de la revendication 1, ladite xylanase étant obtenue à partir d'un organisme sélectionné à partir du groupe constitué de *Schizophyllum commune, Streptomyces lividans, Trichoderma reesei,* et *Trichoderma viride.*


### SEQUENCE LISTING

<110> Wing Dr., Sung L.
   Tolan Dr., Jeffrey S.
<120> Xylanases with Improved Performance in Feed Pelleting Applications
<130> 0888161US
<140>
   <141>
<150> 60/108,504
   <151> 1998-11-16
<160> 61
<170> Patent In Ver. 2.1
<210> 1
   <211> 184
   <212> PRT
   <213> Aspergillus niger
<400> 1
   Ser Ala Gly Ile Asn Tyr Val Gln Asn Tyr Asn Gly Asn Leu Gly Asp
   1 5 10 15
   Phe Thr Tyr Asp Glu Ser Ala Gly Thr Phe Ser Met Tyr Trp Glu Asp
   20 25 30
   Gly Val Ser Ser Asp Phe Val Val Gly Leu Gly Trp Thr Thr Gly Ser
   35 40 45
   Ser Asn Ala Ile Thr Tyr Ser Ala Glu Tyr Ser Ala Ser Gly Ser Ser
   50 55 60
   Ser Tyr Leu Ala Val Tyr Gly Trp Val Asn Tyr Pro Gly Ala Glu Tyr
   65 70 75 80
   Tyr Ile Val Glu Asp Tyr Gly Asp Tyr Asn Pro Cys Ser Ser Ala Thr
   85 90 95
   Ser Leu Gly Thr Val Tyr Ser Asp Gly Ser Thr Tyr Gln Val Cys Thr
   100 105 110
   Asp Thr Arg Ile Asn Glu Pro Ser Ile Thr Gly Thr Ser Thr Phe Thr
   115 120 125
   Gln Tyr Phe Ser Val Arg Glu Ser Thr Arg Thr Ser Gly Thr Val Thr
   130 135 140
   Val Ala Asn His Phe Asn Phe Trp Ala Gln His Gly Phe Gly Asn Ser
   145 150 155 160
   Asp Phe Asn Tyr Gln Val Met Ala Val Glu Ala Trp Ser Gly Ala Gly
   165 170 175
   Ser Ala Ser Val Thr Ile Ser Ser
   180
<210> 2
   <211> 185
   <212> PRT
   <213> Aspergillus tubingensis
<400> 2
   Ser Ala Gly Ile Asn Tyr Val Gln Asn Tyr Asn Gln Asn Leu Gly Asp
   1 5 10 15
   Phe Thr Tyr Asp Glu Ser Ala Gly Thr Phe Ser Met Tyr Trp Glu Asp
   20 25 30
   Gly Val Ser Ser Asp Phe Val Val Gly Leu Gly Gly Trp Thr Thr Gly
   35 40 45
   Ser Ser Asn Ala Ile Thr Tyr Ser Ala Glu Tyr Ser Ala Ser Gly Ser
   50 55 60
   Ala Ser Tyr Leu Ala Val Tyr Gly Trp Val Asn Tyr Pro Gln Ala Glu
   65 70 75 80
   Tyr Tyr Ile Val Glu Asp Tyr Gly Asp Tyr Asn Pro Cys Ser Ser Ala
   85 90 95
   Thr Ser Leu Gly Thr Val Tyr Ser Asp Gly Ser Thr Tyr Gln Val Cys
   100 105 110
   Thr Asp Thr Arg Ile Asn Glu Pro Ser Ile Thr Gly Thr Ser Thr Phe
   115 120 125
   Thr Gln Tyr Phe Ser Val Arg Glu Ser Thr Arg Thr Ser Gly Thr Val
   130 135 140
   Thr Val Ala Asn His Phe Asn Phe Trp Ala His His Gly Phe His Asn
   145 150 155 160
   Ser Asp Phe Asn Tyr Gln Val Val Ala Val Glu Ala Trp Ser Gly Ala
   165 170 175
   Gly Ser Ala Ala Val Thr Ile Ser Ser
   180 185
<210> 3
   <211> 185
   <212> PRT
   <213> Bacillus circulans
<400> 3
   Ala Ser Thr Asp Tyr Trp Gln Asn Trp Thr Asp Gly Gly Gly Ile Val
   1 5 10 15
   Asn Ala Val Asn Gly Ser Gly Gly Asn Tyr Ser Val Asn Trp Ser Asn
   20 25 30
   Thr Gly Asn Phe Val Val Gly Lys Gly Trp Thr Thr Gly Ser Pro Phe
   35 40 45
   Arg Thr Ile Asn Tyr Asn Ala Gly Val Trp Ala Pro Asn Gly Asn Gly
   50 55 60
   Tyr Leu Thr Leu Tyr Gly Trp Thr Arg Ser Pro Leu Ile Glu Tyr Tyr
   65 70 75 80
   Val Val Asp Ser Trp Gly Thr Tyr Arg Pro Thr Gly Thr Tyr Lys Gly
   85 90 95
   Thr Val Lys Ser Asp Gly Gly Thr Tyr Asp Ile Tyr Thr Thr Thr Arg
   100 105 110
   Tyr Asn Ala Pro Ser Ile Asp Gly Asp Arg Thr Thr Phe Thr Gln Tyr
   115 120 125
   Trp Ser Val Arg Gln Ser Lys Arg Pro Thr Gly Ser Asn Ala Thr Ile
   130 135 140
   Thr Phe Thr Asn His Val Asn Ala Trp Lys Ser His Gly Met Asn Leu
   145 150 155 160
   Gly Ser Asn Trp Ala Tyr Gln Val Met Ala Thr Glu Gly Tyr Gln Ser
   165 170 175
   Ser Gly Ser Ser Asn Val Thr Val Trp
   180 185
<210> 4
   <211> 201
   <212> PRT
   <213> Bacillus pumilus
<400> 4
   Arg Thr Ile Thr Asn Asn Glu Met Gly Asn His Ser Gly Tyr Asp Tyr
   1 5 10 15
   Glu Leu Trp Lys Asp Tyr Gly Asn Thr Ser Met Thr Leu Asn Asn Gly
   20 25 30
   Gly Ala Phe Ser Ala Gly Trp Asn Asn Ile Gly Asn Ala Leu Phe Arg
   35 40 45
   Lys Gly Lys Lys Phe Asp Ser Thr Arg Thr His His Gln Leu Gly Asn
   50 55 60
   Ile Ser Ile Asn Tyr Asn Ala Ser Phe Asn Pro Ser Gly Asn Ser Tyr
   65 70 75 80
   Leu Cys Val Tyr Gly Trp Thr Gln Ser Pro Leu Ala Glu Tyr Tyr Ile
   85 90 95
   Val Asp Ser Trp Gly Thr Tyr Arg Pro Thr Gly Ala Tyr Lys Gly Ser
   100 105 110
   Phe Tyr Ala Asp Gly Gly Thr Tyr Asp Ile Tyr Glu Thr Thr Arg Val
   115 120 125
   Asn Gln Pro Ser Ile Ile Gly Ile Ala Thr Phe Lys Gln Tyr Trp Ser
   130 135 140
   Val Arg Gln Thr Lys Arg Thr Ser Gly Thr Val Ser Val Ser Ala His
   145 150 155 160
   Phe Arg Lys Trp Glu Ser Leu Gly Met Pro Met Gly Lys Met Tyr Glu
   165 170 175
   Thr Ala Phe Thr Val Glu Gly Tyr Gln Ser Ser Gly Ser Ala Asn Val
   180 185 190
   Met Thr Asn Gln Leu Phe Ile Gly Asn
   195 200
<210> 5
   <211> 185
   <212> PRT
   <213> Bacillus subtilis
<210> 6
   <211> 211
   <212> PRT
   <213> Clostridium acetobutylicum
<400> 6
   Ser Ala Phe Asn Thr Gln Ala Ala Pro Lys Thr Ile Thr Ser Asn Glu
   1 5 10 15
   Ile Gly Val Asn Gly Gly Tyr Asp Tyr Glu Leu Trp Lys Asp Tyr Gly
   20 25 30
   Asn Thr Ser Met Thr Leu Lys Asn Gly Gly Ala Phe Ser Cys Gln Trp
   35 40 45
   Ser Asn Ile Gly Asn Ala Leu Phe Arg Lys Gly Lys Lys Phe Asn Asp
   50 55 60
   Thr Gln Thr Tyr Lys Gln Leu Gly Asn Ile Ser Val Asn Tyr Asn Cys
   65 70 75 80
   Asn Tyr Gln Pro Tyr Gly Asn Ser Tyr Leu Cys Val Tyr Gly Trp Thr
   85 90 95
   Ser Ser Pro Leu Val Glu Tyr Tyr Ile Val Asp Ser Trp Gly Ser Trp
   100 105 110
   Arg Pro Pro Gly Gly Thr Ser Lys Gly Thr Ile Thr Val Asp Gly Gly
   115 120 125
   Ile Tyr Asp Ile Tyr Glu Thr Thr Arg Ile Asn Gln Pro Ser Ile Gln
   130 135 140
   Gly Asn Thr Thr Phe Lys Gln Tyr Trp Ser Val Arg Arg Thr Lys Arg
   145 150 155 160
   Thr Ser Gly Thr Ile Ser Val Ser Lys His Phe Ala Ala Trp Glu Ser
   165 170 175
   Lys Gly Met Pro Leu Gly Lys Met His Glu Thr Ala Phe Asn Ile Glu
   180 185 190
   Gly Tyr Gln Ser Ser Gly Lys Ala Asp Val Asn Ser Met Ser Ile Asn
   195 200 205
   Ile Gly Lys
   210
<210> 7
   <211> 206
   <212> PRT
   <213> Clostridium stercorarium
<400> 7
   Gly Arg Ile Ile Tyr Asp Asn Glu Thr Gly Thr His Gly Gly Tyr Asp
   1 5 10 15
   Tyr Glu Leu Trp Lys Asp Tyr Gly Asn Thr Ile Met Glu Leu Asn Asp
   20 25 30
   Gly Gly Thr Phe Ser Cys Gln Trp Ser Asn Ile Gly Asn Ala Leu Phe
   35 40 45
   Arg Lys Gly Arg Lys Phe Asn Ser Asp Lys Thr Tyr Gln Glu Leu Gly
   50 55 60
   Asp Ile Val Val Glu Tyr Gly Cys Asp Tyr Asn Pro Asn Gly Asn Ser
   65 70 75 80
   Tyr Leu Cys Val Tyr Gly Trp Thr Arg Asn Phe Leu Val Glu Tyr Tyr
   85 90 95
   Ile Val Glu Ser Trp Gly Ser Trp Arg Pro Pro Gly Ala Thr Pro Lys
   100 105 110
   Gly Thr Ile Thr Gln Trp Met Ala Gly Thr Tyr Glu Ile Tyr Glu Thr
   115 120 125
   Thr Arg Val Asn Gln Pro Ser Ile Asp Gly Thr Ala Thr Phe Gln Gln
   130 135 140
   Tyr Trp Ser Val Arg Thr Ser Lys Arg Thr Ser Gly Thr Ile Ser Val
   145 150 155 160
   Thr Glu His Phe Lys Gln Trp Glu Arg Met Gly Met Arg Met Gly Lys
   165 170 175
   Met Tyr Glu Val Ala Leu Thr Val Glu Gly Tyr Gln Ser Ser Gly Tyr
   180 185 190
   Ala Asn Val Tyr Lys Asn Glu Ile Arg Ile Gly Ala Asn Pro
   195 200 205
<210> 8
   <211> 211
   <212> PRT
   <213> Ruminococcus flavefaciens
<400> 8
   Ser Ala Ala Asp Gln Gln Thr Arg Gly Asn Val Gly Gly Tyr Asp Tyr
   1 5 10 15
   Glu Met Trp Asn Gln Asn Gly Gln Gly Gln Ala Ser Met Asn Pro Gly
   20 25 30
   Ala Gly Ser Phe Thr Cys Ser Trp Ser Asn Ile Glu Asn Phe Leu Ala
   35 40 45
   Arg Met Gly Lys Asn Tyr Asp Ser Gln Lys Lys Asn Tyr Lys Ala Phe
   50 55 60
   Gly Asn Ile Val Leu Thr Tyr Asp Val Glu Tyr Thr Pro Arg Gly Asn
   65 70 75 80
   Ser Tyr Met Cys Val Tyr Gly Trp Thr Arg Asn Pro Leu Met Glu Tyr
   85 90 95
   Tyr Ile Val Glu Gly Trp Gly Asp Trp Arg Pro Pro Gly Asn Asp Gly
   100 105 110
   Glu Val Lys Gly Thr Val Ser Ala Asn Gly Asn Thr Tyr Asp Ile Arg
   115 120 125
   Lys Thr Met Arg Tyr Asn Gln Pro Ser Leu Asp Gly Thr Ala Thr Phe
   130 135 140
   Pro Gln Tyr Trp Ser Val Arg Gln Thr Ser Gly Ser Ala Asn Asn Gln
   145 150 155 160
   Thr Asn Tyr Met Lys Gly Thr Ile Asp Val Ser Lys His Phe Asp Ala
   165 170 175
   Trp Ser Ala Ala Gly Leu Asp Met Ser Gly Thr Leu Tyr Glu Val Ser
   180 185 190
   Leu Asn Ile Glu Gly Tyr Arg Ser Asn Gly Ser Ala Asn Val Lys Ser
   195 200 205
   Val Ser Val
   210
<210> 9
   <211> 197
   <212> PRT
   <213> Schizophyllum commune
<400> 9
   Ser Gly Thr Pro Ser Ser Thr Gly Thr Asp Gly Gly Tyr Tyr Tyr Ser
   1 5 10 15
   Trp Trp Thr Asp Gly Ala Gly Asp Ala Thr Tyr Gln Asn Asn Gly Gly
   20 25 30
   Gly Ser Tyr Thr Leu Thr Trp Ser Gly Asn Asn Gly Asn Leu Val Gly
   35 40 45
   Gly Lys Gly Trp Asn Pro Gly Ala Ala Ser Arg Ser Ile Ser Tyr Ser
   50 55 60
   Gly Thr Tyr Gln Pro Asn Gly Asn Ser Tyr Leu Ser Val Tyr Gly Trp
   65 70 75 80
   Thr Arg Ser Ser Leu Ile Glu Tyr Tyr Ile Val Glu Ser Tyr Gly Ser
   85 90 95
   Tyr Asp Pro Ser Ser Ala Ala Ser His Lys Gly Ser Val Thr Cys Asn
   100 105 110
   Gly Ala Thr Tyr Asp Ile Leu Ser Thr Trp Arg Tyr Asn Ala Pro Ser
   115 120 125
   Ile Asp Gly Thr Gln Thr Phe Glu Gln Phe Trp Ser Val Arg Asn Pro
   130 135 140
   Lys Lys Ala Pro Gly Gly Ser Ile Ser Gly Thr Val Asp Val Gln Cys
   145 150 155 160
   His Phe Asp Ala Trp Lys Gly Leu Gly Met Asn Leu Gly Ser Glu His
   165 170 175
   Asn Tyr Gln Ile Val Ala Thr Glu Gly Tyr Gln Ser Ser Gly Thr Ala
   180 185 190
   Thr Ile Thr Val Thr
   195
<210> 10
   <211> 191
   <212> PRT
   <213> Streptomyces lividans
<210> 11
   <211> 191
   <212> PRT
   <213> Streptomyces lividans
<400> 11
   Ala Thr Thr Ile Thr Thr Asn Gln Thr Gly Thr Asp Gly Met Tyr Tyr
   1               5                   10                  15
   Ser Phe Trp Thr Asp Gly Gly Gly Ser Val Ser Met Thr Leu Asn Gly
               20                  25                  30
   Gly Gly Ser Tyr Ser Thr Gln Trp Thr Asn Cys Gly Asn Phe Val Ala
           35                  40                  45
   Gly Lys Gly Trp Ser Thr Gly Asp Gly Asn Val Arg Tyr Asn Gly Tyr
       50                  55                  60
   Phe Asn Pro Val Gly Asn Gly Tyr Gly Cys Leu Tyr Gly Trp Thr Ser
   65                  70                  75                  80
   Asn Pro Leu Val Glu Tyr Tyr Ile Val Asp Asn Trp Gly Ser Tyr Arg
                   85                  90                  95
   Pro Thr Gly Thr Tyr Lys Gly Thr Val Ser Ser Asp Gly Gly Thr Tyr
               100                 105                 110
   Asp Ile Tyr Gln Thr Thr Arg Tyr Asn Ala Pro Ser Val Glu Gly Thr
           115                 120                 125
   Lys Thr Phe Gln Gln Tyr Trp Ser Val Arg Gln Ser Lys Val Thr Ser
       130                 135                 140
   Gly Ser Gly Thr Ile Thr Thr Gly Asn His Phe Asp Ala Trp Ala Arg
   145                 150                 155                 160
   Ala Gly Met Asn Met Gly Gln Phe Arg Tyr Tyr Met Ile Asn Ala Thr
                   165                 170                 175
   Glu Gly Tyr Gln Ser Ser Gly Ser Ser Asn Ile Thr Val Ser Gly
               180                 185                 190
<210> 12
   <211> 189
   <212> PRT
   <213> Streptomyces sp.
<400> 12
   Ala Thr Thr Ile Thr Asn Glu Thr Gly Tyr Asp Gly Met Tyr Tyr Ser
   1 5 10 15
   Phe Trp Thr Asp Gly Gly Gly Ser Val Ser Met Thr Leu Asn Gly Gly
   20 25 30
   Gly Ser Tyr Ser Thr Arg Trp Thr Asn Cys Gly Asn Phe Val Ala Gly
   35 40 45
   Lys Gly Trp Ala Asn Gly Gly Arg Arg Thr Val Arg Tyr Thr Gly Trp
   50 55 60
   Phe Asn Pro Ser Gly Asn Gly Tyr Gly Cys Leu Tyr Gly Trp Thr Ser
   65 70 75 80
   Asn Pro Leu Val Glu Tyr Tyr Ile Val Asp Asn Trp Gly Ser Tyr Arg
   85 90 95
   Pro Thr Gly Glu Thr Arg Gly Thr Val His Ser Asp Gly Gly Thr Tyr
   100 105 110
   Asp Ile Tyr Lys Thr Thr Arg Tyr Asn Ala Pro Ser Val Glu Ala Pro
   115 120 125
   Ala Ala Phe Asp Gln Tyr Trp Ser Val Arg Gln Ser Lys Val Thr Ser
   130 135 140
   Gly Thr Ile Thr Thr Gly Asn His Phe Asp Ala Trp Ala Arg Ala Gly
   145 150 155 160
   Met Asn Met Gly Asn Phe Arg Tyr Tyr Met Ile Asn Ala Thr Glu Gly
   165 170 175
   Tyr Gln Ser Ser Gly Ser Ser Thr Ile Thr Val Ser Gly
   180 185
<210> 13
   <211> 189
   <212> PRT
   <213> Thermomonospora fusca
<400> 13
   Ala Val Thr Ser Asn Glu Thr Gly Tyr His Asp Gly Tyr Phe Tyr Ser
   1 5 10 15
   Phe Trp Thr Asp Ala Pro Gly Thr Val Ser Met Glu Leu Gly Pro Gly
   20 25 30
   Gly Asn Tyr Ser Thr Ser Trp Arg Asn Thr Gly Asn Phe Val Ala Gly
   35 40 45
   Lys Gly Trp Ala Thr Gly Gly Arg Arg Thr Val Thr Tyr Ser Ala Ser
   50 55 60
   Phe Asn Pro Ser Gly Asn Ala Tyr Leu Thr Leu Tyr Gly Trp Thr Arg
   65 70 75 80
   Asn Pro Leu Val Glu Tyr Tyr Ile Val Glu Ser Trp Gly Thr Tyr Arg
   85 90 95
   Pro Thr Gly Thr Tyr Met Gly Thr Val Thr Thr Asp Gly Gly Thr Tyr
   100 105 110
   Asp Ile Tyr Lys Thr Thr Arg Tyr Asn Ala Pro Ser Ile Glu Gly Thr
   115 120 125
   Arg Thr Phe Asp Gln Tyr Trp Ser Val Arg Gln Ser Lys Arg Thr Ser
   130 135 140
   Gly Thr Ile Thr Ala Gly Asn His Phe Asp Ala Trp Ala Arg His Gly
   145 150 155 160
   Met His Leu Gly Thr His Asp Tyr Met Ile Met Ala Thr Glu Gly Tyr
   165 170 175
   Gln Ser Ser Gly Ser Ser Asn Val Thr Leu Gly Thr Ser
   180 185
<210> 14
   <211> 190
   <212> PRT
   <213> Trichoderma harzianum
<400> 14
   Gln Thr Ile Gly Pro Gly Thr Gly Tyr Ser Asn Gly Tyr Tyr Tyr Ser
   1 5 10 15
   Tyr Trp Asn Asp Gly His Ala Gly Val Thr Tyr Thr Asn Gly Gly Gly
   20 25 30
   Gly Ser Phe Thr Val Asn Trp Ser Asn Ser Gly Asn Phe Val Gly Gly
   35 40 45
   Lys Gly Trp Gln Pro Gly Thr Lys Asn Lys Val Ile Asn Phe Ser Gly
   50 55 60
   Ser Tyr Asn Pro Asn Gly Asn Ser Tyr Leu Ser Ile Tyr Gly Trp Ser
   65 70 75 80
   Arg Asn Pro Leu Ile Glu Tyr Tyr Ile Val Glu Asn Phe Gly Thr Tyr
   85 90 95
   Asn Pro Ser Thr Gly Ala Thr Lys Leu Gly Glu Val Thr Ser Asp Gly
   100 105 110
   Ser Val Tyr Asp Ile Tyr Arg Thr Gln Arg Val Asn Gln Pro Ser Ile
   115 120 125
   Ile Gly Thr Ala Thr Phe Tyr Gln Tyr Trp Ser Val Arg Arg Asn His
   130 135 140
   Arg Ser Ser Gly Ser Val Asn Thr Ala Asn His Phe Asn Ala Trp Ala
   145 150 155 160
   Ser His Gly Leu Thr Leu Gly Thr Met Asp Tyr Gln Ile Val Ala Val
   165 170 175
   Glu Gly Tyr Phe Ser Ser Gly Ser Ala Ser Ile Thr Val Ser
   180 185 190
<210> 15
   <211> 178
   <212> PRT
   <213> Trichoderma reesei
<400> 15
   Ala Ser Ile Asn Tyr Asp Gln Asn Tyr Gln Thr Gly Gly Gln Val Ser
   1 5 10 15
   Tyr Ser Pro Ser Asn Thr Gly Phe Ser Val Asn Trp Asn Thr Gln Asp
   20 25 30
   Asp Phe Val Val Gly Val Gly Trp Thr Thr Gly Ser Ser Ala Pro Ile
   35 40 45
   Asn Phe Gly Gly Ser Phe Ser Val Asn Ser Gly Thr Gly Leu Leu Ser
   50 55 60
   Val Tyr Gly Trp Ser Thr Asn Pro Leu Val Glu Tyr Tyr Ile Met Glu
   65 70 75 80
   Asp Asn His Asn Tyr Pro Ala Gln Gly Thr Val Lys Gly Thr Val Thr
   85 90 95
   Ser Asp Gly Ala Thr Tyr Thr Ile Trp Glu Asn Thr Arg Val Asn Glu
   100 105 110
   Pro Ser Ile Gln Gly Thr Ala Thr Phe Asn Gln Tyr Ile Ser Val Arg
   115 120 125
   Asn Ser Pro Arg Thr Ser Gly Thr Val Thr Val Gln Asn His Phe Asn
   130 135 140
   Trp Ala Ser Leu Gly Leu His Leu Gly Gln Met Met Asn Tyr Gln Val
   145 150 155 160
   Val Ala Val Glu Gly Trp Gly Gly Ser Gly Ser Ala Ser Gln Ser Val
   165 170 175
   Ser Asn
<210> 16
   <211> 190
   <212> PRT
   <213> Trichoderma reesei
<400> 16
   Gln Thr Ile Gln Pro Gly Thr Gly Tyr Asn Asn Gly Tyr Phe Tyr Ser
   1 5 10 15
   Tyr Trp Asn Asp Gly His Gly Gly Val Thr Tyr Thr Asn Gly Pro Gly
   20 25 30
   Gly Gln Phe Ser Val Asn Trp Ser Asn Ser Gly Asn Phe Val Gly Gly
   35 40 45
   Lys Gly Trp Gln Pro Gly Thr Lys Asn Lys Val Ile Asn Phe Ser Gly
   50 55 60
   Ser Tyr Asn Pro Asn Gly Asn Ser Tyr Leu Ser Val Tyr Gly Trp Ser
   65 70 75 80
   Arg Asn Pro Leu Ile Glu Tyr Tyr Ile Val Glu Asn Phe Gly Thr Tyr
   85 90 95
   Asn Pro Ser Thr Gly Ala Thr Lys Leu Gly Glu Val Thr Ser Asp Gly
   100 105 110
   Ser Val Tyr Asp Ile Tyr Arg Thr Gln Arg Val Asn Gln Pro Ser Ile
   115 120 125
   Ile Gly Thr Ala Thr Phe Tyr Gln Tyr Trp Ser Val Arg Arg Asn His
   130 135 140
   Arg Ser Ser Gly Ser Val Asn Thr Ala Asn His Phe Asn Ala Trp Ala
   145 150 155 160
   Gln Gln Gly Leu Thr Leu Gly Thr Met Asp Tyr Gln Ile Val Ala Val
   165 170 175
   Glu Gly Tyr Phe Ser Ser Gly Ser Ala Ser Ile Thr Val Ser
   180 185 190
<210> 17
   <211> 190
   <212> PRT
   <213> Trichoderma viride
<400> 17
   Gln Thr Ile Gln Pro Gly Thr Gly Phe Asn Asn Gly Tyr Phe Tyr Ser
   1               5                   10                  15
   Tyr Trp Asn Asp Gly His Gly Gly Val Thr Tyr Thr Asn Gly Pro Gly
               20                  25                  30
   Gly Gln Phe Ser Val Asn Trp Ser Asn Ser Gly Asn Phe Val Gly Gly
           35                  40                  45
   Lys Gly Trp Gln Pro Gly Thr Lys Asn Lys Val Ile Asn Phe Ser Gly
       50                  55                  60
   Ser Tyr Asn Pro Asn Gly Asn Ser Tyr Leu Ser Val Tyr Gly Trp Ser
   65                  70                  75                  80
   Arg Asn Pro Leu Ile Glu Tyr Tyr Ile Val Glu Asn Phe Gly Thr Tyr
                   85                  90                  95
   Asn Pro Ser Thr Gly Ala Thr Lys Leu Gly Glu Val Thr Ser Asp Gly
               100                 105                 110
   Ser Val Tyr Asp Ile Tyr Arg Thr Gln Arg Val Asn Gln Pro Ser Ile
           115                 120                 125
   Ile Gly Thr Ala Thr Phe Tyr Gln Tyr Trp Ser Val Arg Arg Thr His
       130                 135                 140
   Arg Ser Ser Gly Ser Val Asn Thr Ala Asn His Phe Asn Ala Trp Ala
   145                 150                 155                 160
   Gln Gln Gly Leu Thr Leu Gly Thr Met Asp Tyr Gln Ile Val Ala Val
                   165                 170                 175
   Glu Gly Tyr Phe Ser Ser Gly Ser Ala Ser Ile Thr Val Ser
               180                 185                 190
<210> 18
   <211> 596
   <212> DNA
   <213> Artificial Sequence
<220>
   <223> Description of Artificial Sequence:TrX synthetic sequence
<400> 18
   ctagctaagg aggctgcaga tgcaaacaat acaaccagga accggttaca acaacggtta 60
   cttttacagc tattggaacg atggccatgg tggtgttacc tatacaaacg ggcccggagg 120
   ccaatttagc gtcaattggt ctaactccgg aaacttcgta ggtggaaaag gttggcaacc 180
   cgggaccaaa aataaggtga tcaacttctc tggatcttat aatccgaatg ggaattcata 240
   cttaagcgtc tatggctggt ctagaaaccc actgattgaa tattacattg tcgaaaattt 300
   cggtacctac aatccgagta ccggcgccac aaaattaggc gaagtcacta gtgatggatc 360
   cgtatatgat atctaccgta cccaacgcgt taatcagcca tcgatcattg gaaccgccac 420
   cttttatcag tactggagtg ttagacgtaa tcatcggagc tccggttcgg ttaatactgc 480
   gaatcacttt aatgcatggg cacagcaagg gttaacccta ggtacaatgg attatcaaat 540
   cgtagcggtg gaaggctacc tctcgagtgg ttccgctagt attacagtga gctaaa     596
<210> 19
   <211> 40
   <212> DNA
   <213> Artificial Sequence
<220>
   <223> Description of Artificial Sequence:Trx-110C Synthetic Sequence
<400> 19
   atatacggat ccatcacaag tgacttcgcc taattttgtg 40
<210> 20
   <211> 68
   <212> DNA
   <213> Artificial Sequence
<220>
   <223> Description of Artificial Sequence:Tx-110C-2
<400> 20
   gcgccacaaa attaggcgaa gtcacttgtg atggatccgt atatgatatc taccgtaccc 60
   aacgcgtt 68
<210> 21
   <211> 42
   <212> DNA
   <213> Artificial Sequence
<220>
   <223> Description of Artificial Sequence:Tx-103b
<400> 21
   aatcagccat cgatcattgg aaccgccacc ttttatcagt ac 42
<210> 22
   <211> 54
   <212> DNA
   <213> Artificial Sequence
<220>
   <223> Description of Artificial Sequence:XyTv-109
   Synthetic sequence
<400> 22
   ggtggcggtt ccaatgatcg atggctgatt aacgcgttgg gtacggtaga tatc 54
<210> 23
   <211> 48
   <212> DNA
   <213> Artificial Sequence
<220>
   <223> Description of Artificial Sequence:Tx-108b
<400> 23
   cgaaccggag ctccgatgat tacgtctaac actccagtac tgataaaa 48
<210> 24
   <211> 52
   <212> DNA
   <213> Artificial Sequence
<220>
   <223> Description of Artificial Sequence:Tx-154C
   Synthetic sequence
<400> 24
   ctagggttaa cccttgtgat gcccaggcat taaagtggca tgcagtatta ac 52
<210> 25
   <211> 84
   <212> DNA
   <213> Artificial Sequence
<220>
   <223> Description of Artificial Sequence:Tx-154C-2
<400> 25
   tggagtgtta gacgtaatca tcggagctcc ggttcggtta atactgcatg ccactttaat 60
   gcctgggcac agcaagggtt aacc 84
<210> 26
   <211> 34
<212> DNA
   <213> Artificial Sequence
<220>
   <223> Description of Artificial Sequence:Tx-162H-3
<400> 26
   ccacttcaat gcatgggcac agcacgggtt aacc 34
<210> 27
   <211> 42
   <212> DNA
   <213> Artificial Sequence
<220>
   <223> Description of Artificial Sequence:TrX-162H-4
<400> 27
   ctagggttaa cccgtgctgt gcccatgcat tgaagtggca tg 42
<210> 28
   <211> 58
   <212> DNA
   <213> Artificial Sequence
<220>
   <223> Description of Artificial Sequence:XyTv-101
<400> 28
   tcgacaattt cggtacctac aatccgagca ccggcgccac aaaattaggc gaagtcac 58
<210> 29
   <211> 52
   <212> DNA
   <213> Artificial Sequence
<220>
   <223> Description of Artificial Sequence :XyTv-102
<400> 29
   tagtgatgga tccgtatatg atatctaccg tacccaacgc gttaatcagc ca 52
<210> 30
   <211> 60
   <212> DNA
   <213> Artificial Sequence
<220>
   <223> Description of Artificial Sequence:TrX-103
<400> 30
   tcgatcattg gaaccgccac cttttatcag tactggagtg ttagacgtaa tcatcggagc 60
<210> 31
   <211> 69
   <212> DNA
   <213> Artificial Sequence
<220>
   <223> Description of Artificial Sequence:XyTv-104
<400> 31
   tccggttcgg ttaatactgc gaatcacttt aatgcatggg cacagcaagg gttaacccta 60
   ggtacaatg 69
<210> 32
   <211> 67
   <212> DNA
   <213> Artificial Sequence
<220>
   <223> Description of Artificial Sequence:XyTv-105
<400> 32
   gattatcaaa tcgtagcggt ggaaggctac ttctcgagtg gttccgctag tattacagtg 60
   agctaaa 67
<210> 33
   <211> 53
   <212> DNA
   <213> Artificial Sequence
<220>
   <223> Description of Artificial Sequence:XyTv-106 synthetic sequence
<400> 33
   gatctttagc tcactgtaat actagcggaa ccactcgaga agtagccttc cac 53
<210> 34
   <211> 66
   <212> DNA
   <213> Artificial Sequence
<220>
   <223> Description of Artificial Sequence:XyTv-107
<400> 34
   cgctacgatt tgataatcca ttgtacctag ggttaaccct tgctgtgccc atgcattaaa 60
   gtgatt 66
<210> 35
   <211> 60
   <212> DNA
   <213> Artificial Sequence.
<220>
   <223> Description of Artificial Sequence:TrX-108
<400> 35
   cgcagtatta accgaaccgg agctccgatg attacgtcta acactccagt actgataaaa 60
<210> 36
   <211> 73
   <212> DNA
   <213> Artificial Sequence
<220>
   <223> Description of Artificial Sequence:XyTv-110
<400> 36
   atatacggat ccatcactag tgacttcgcc taattttgtg gcgccggtac tcggattgta 60
   ggtaccgaaa ttg 73
<210> 37
   <211> 76
   <212> DNA
   <213> Artificial Sequence
<220>
   <223> Description of Artificial Sequence:TrX-1
<400> 37
   ctagctaagg aggctgcaga tgcaaacaat acaaccagga accggttaca acaacggtta 60
   cttttacagc tattgg 76
<210> 38
   <211> 78
   <212> DNA
   <213> Artificial Sequence
   <220>
   <223> Description of Artificial Sequence:XyTv-2
<400> 38
   aacgatggcc atggtggtgt tacctataca aacgggcccg gaggccaatt tagcgtcaat 60
   tggtctaact ccggaaac 78
<210> 39
   <211> 78
   <212> DNA
   <213> Artificial Sequence
<220>
   <223> Description of Artificial Sequence:TrX-3
<400> 39
   ttcgtaggtg gaaaaggttg gcaacccggg accaaaaata aggtgatcaa cttctctgga 60
   tcttataatc cgaatggg 78
<210> 40
   <211> 74
   <212> DNA
   <213> Artificial Sequence
<220>
   <223> Description of Artificial Sequence:XyTv-4
<400> 40
   aattcatact taagcgtcta tggctggtct agaaacccac tgattgaata ttacattgtc 60
   gaaaatttcg gtac 74
<210> 41
   <211> 85
   <212> DNA
   <213> Artificial Sequence
<220>
   <223> Description of Artificial Sequence :XyTv-5
<400> 41
   gcaaattttc gacaatgtaa tattcaatca gtgggtttct agaccagcca tagacgctta 60
   agtatgaatt cccattcgga ttata 85
<210> 42
   <211> 78
   <212> DNA
   <213> Artificial Sequence
<220>
   <223> Description of Artificial Sequence:Trx-6Synthetic sequence
<400> 42
   agatccagag aagttgatca ccttattttt ggtcccgggt tgccaacctt ttccacctac 60
   gaagtttccg gagttaga 78
<210> 43
   <211> 84
   <212> DNA
   <213> Artificial Sequence
<220>
   <223> Description of Artificial Sequence:XyTv-7
   Synthetic sequence
<400> 43
   ccaattgacg ctaaattggc ctccgggccc gtttgtatag gtaacaccac catggccatc 60
   gttccaatag ctgtaaaagt aacc 84
<210> 44
   <211> 51
   <212> DNA
   <213> Artificial Sequence
<220>
   <223> Description of Artificial Sequence:TrX-8 synthetic
   sequence
<400> 44
   gttgttgtaa ccggttcctg gttgtattgt ttgcatctgc agcctcctta g 51
<210> 45
   <211> 40
   <212> DNA
   <213> Artificial Sequence
<220>
   <223> Description of Artificial Sequence:Tx-108C
   synthetic sequence
<400> 45
   atatacggat ccatcactag tgcattcgcc taattttgtg 40
<210> 46
   <211> 68
   <212> DNA
   <213> Artificial Sequence
<220>
   <223> Description of Artificial Sequence:Tx-108C-2
<400> 46
   gcgccacaaa attaggcgaa tgcactagtg atggatccgt atatgatatc taccgtaccc 60
   aacgcgtt 68
<210> 47
   <211> 52
   <212> DNA
   <213> Artificial Sequence
<220>
   <223> Description of Artificial Sequence:Tx-158C-162H
   synthetic sequence
<400> 47
   ctagggttaa cccgtgtgat gcccagcaat taaagtgatt tgcagtatta ac 52
<210> 48
   <211> 84
   <212> DNA
   <213> Artificial Sequence
<220>
   <223> Description of Artificial Sequence:TX-158C-162H-2
<400> 48
   tggagtgtta gacgtaatca tcggagctcc ggttcggtta atactgcaaa tcactttaat 60
   tgctgggcac agcacgggtt aacc 84
<210> 49
   <211> 40
   <212> DNA
   <213> Artificial Sequence
<220>
   <223> Description of Artificial Sequence:Tx-108c-110c
   synthetic seqeuence
<400> 49
   atatacggat ccatcacaag tgcattcgcc taattttgtg 40
<210> 50
   <211> 68
   <212> DNA
   <213> Artificial Sequence
<220>
   <223> Description of Artificial Sequence:Tx-108C-110C-2
   synthetic sequence
<400> 50
   gcgccacaaa attaggcgaa tgcacttgtg atggatccgt atatgatatc taccgtaccc 60
   aacgcgtt 68
<210> 51
   <211> 52
   <212> DNA
   <213> Artificial Sequence
<220>
   <223> Description of Artificial
   Sequence:Tx-154C-15SC-152H synthetic seqeunce
<400> 51
   ctagggttaa cccgtgtgat gcccagcaat taaagtggca tgcagtatta ac 52
<210> 52
   <211> 84
   <212> DNA
   <213> Artificial Sequence
<220>
   <223> Description of Artificial
   Sequence:Tx-154C-158C-162H-2
<400> 52
   tggagtgtta gacgtaatca tcggagctcc ggttcggtta atactgcatg ccactttaat 60
   tgctgggcac agcacgggtt aacc 84
<210> 53
   <211> 190
   <212> PRT
   <213> Artificial Sequence
<220>
   <223> Description of Artificial Sequence: TrX amino acid
   sequence
<400> 53.
   Gln Thr Ile Gln Pro Gly Thr Gly Tyr Asn Asn Gly Tyr Phe Tyr Ser
   1 5 10 15
   Tyr Trp Asn Asp Gly His Gly Gly Val Thr Tyr Thr Asn Gly Pro Gly
   20 25 30
   Gly Gln Phe Ser Val Asn Trp Ser Asn Ser Gly Asn Phe Val Gly Gly
   35 40 45
   Lys Gly Trp Gln Pro Gly Thr Lys Asn Lys Val Ile Asn Phe Ser Gly
   50 55 60
   Ser Tyr Asn Pro Asn Gly Asn Ser Tyr Leu Ser Val Tyr Gly Trp Ser
   65 70 75 80
   Arg Asn Pro Leu Ile Glu Tyr Tyr Ile Val Glu Asn Phe Gly Thr Tyr
   85 90 95
   Asn Pro Ser Thr Gly Ala Thr Lys Leu Gly Glu Val Thr Ser Asp Gly
   100 105 110
   Ser Val Tyr Asp Ile Tyr Arg Thr Gln Arg Val Asn Gln Pro Ser Ile
   115 120 125
   Ile Gly Thr Ala Thr Phe Tyr Gln Tyr Trp Ser Val Arg Arg Asn His
   130 135 140
   Arg Ser Ser Gly Ser Val Asn Thr Ala Asn His Phe Asn Ala Trp Ala
   145 150 155 160
   Gln Gln Gly Leu Thr Leu Gly Thr Met Asp Tyr Gln Ile Val Ala Val
   165 170 175
   Glu Gly Tyr Phe Ser Ser Gly Ser Ala Ser Ile Thr Val Ser
   180 185 190
<210> 54
   <211> 198
   <212> DNA
   <213> Artificial Sequence
<220>
   <223> Description of Artificial Sequence:TrX-DS1
   cassette
<400> 54
   gcgccacaaa attaggcgaa gtcacttgtg atggatccgt atatgatatc taccgtaccc 60
   aacgcgttaa tcagccatcg atcattggaa ccgccacctt ttatcagtac tggagtgtta 120
   gacgtaatca tcggagctcc ggttcggtta atactgcatg ccactttaat gcctgggcac 180
   agcaagggtt aaccctag 198
<210> 55
   <211> 67
   <212> PRT
   <213> Artificial Sequence
<220>
   <223> Description of Artificial sequence:TrX-DS1
   cassette aa
<210> 56
   <211> 15
   <212> PRT
   <213> Artificial Sequence
<220>
   <223> Description of Artificial Sequence:TrX-162H-DS1
   cassette aa
<400> 56
   Ala Cys His Phe Asn Ala Trp Ala Gln His Gly Leu Thr Leu Gly
   1               5                   10                  15
<210> 57
   <211> 198
   <212> DNA
   <213> Artificial Sequence
<220>
   <223> Description of Artificial Sequence:TrX-162H-DS2
   cassette
<400> 57
   gcgccacaaa attaggcgaa tgcactagtg atggatccgt atatgatatc taccgtaccc 60
   aacgcgttaa tcagccatcg atcattggaa ccgccacctt ttatcagtac tggagtgtta 120
   gacgtaatca tcggagctcc ggttcggtta atactgcaaa tcactttaat tgctgggcac 180
   agcacgggtt aaccctag 198
<210> 58
   <211> 67
   <212> PRT
   <213> Artificial Sequence
<220>
   <223> Description of Artificial Sequence:TrX-162H-DS2
   cassette aa
   <400> 58
   Gly Ala Thr Lys Leu Gly Glu Cys Thr Ser Asp Ser Ser Val Tyr Asp
   1               5                   10                  15
   Ile Tyr Arg Thr Gln Arg Val Asn Gln Pro Ser Ile Ile Gly Thr Ala
               20                  25                  30
   Thr Phe Tyr Gln Tyr Trp Ser Val Arg Arg Asn His Arg Ser Ser Gly
           35                  40                  45
   Ser Val Asn Thr Ala Asn His Phe Asn Cys Trp Ala Gln His Gly Leu
       50                  55                  60
   Thr Leu Gly
   65
<210> 59
   <211> 198
   <212> DNA
   <213> Artificial Sequence
<220>
   <223> Description of Artificial Sequence:TrX-162H-DS4
   cassette
   <400> 59
   gcgccacaaa attaggcgaa tgcacttgtg atggatccgt atatgatatc taccgtaccc 60
   aacgcgttaa tcagccatcg atcattggaa ccgccacctt ttatcagtac tggagtgtta 120
   gacgtaatca tcggagctcc ggttcggtta atactgcatg ccactttaat tgctgggcac 180
   agcacgggtt aaccctag 198
<210> 60
   <211> 67
   <212> PRT
   <213> Artificial Sequence
<220>
   <223> Description of Artificial Sequence:TrX-162H-DS4
   cassete aa
   <400> 60
   Gly Ala Thr Lys Leu Gly Glu Cys Thr Cys Asp Gly Ser Val Tyr Asp
   1               5                   10                  15
   Ile Tyr Arg Thr Gln Arg Val Asn Gln Pro Ser Ile Ile Gly Thr Ala
               20                  25                  30
   Thr Phe Tyr Gln Tyr Trp Ser Val Arg Arg Asn His Arg Ser Ser Gly
   35 40 45
   Ser Val Asn Thr Ala Cys His Phe Asn Cys Trp Ala Gln His Gly Leu
   50 55 60
   Thr Leu Gly
   65
<210> 61
   <211> 42
   <212> DNA
   <213> Artificial Sequence
<220>
   <223> Description of Artificial Sequence:TrX-162H-DS1
   cassette
<400> 61
   catgccactt caatgcatgg gcacagcacg ggttaaccct ag 42